(19) Europäisches Patentamt
European Patent Office
Office européen des brevets

(11) EP 3 429 995 B1

(12) # EUROPÄISCHE PATENTSCHRIFT

(45) Veröffentlichungstag und Bekanntmachung des Hinweises auf die Patenterteilung:
**07.10.2020 Patentblatt 2020/41**

(21) Anmeldenummer: **17716468.8**

(22) Anmeldetag: **16.03.2017**

(51) Int Cl.:
***C07D 209/86*** *(2006.01)* ***H01L 51/00*** *(2006.01)*
***H01L 51/50*** *(2006.01)*

(86) Internationale Anmeldenummer:
**PCT/EP2017/056226**

(87) Internationale Veröffentlichungsnummer:
**WO 2017/158083 (21.09.2017 Gazette 2017/38)**

(54) **ORGANISCHE MOLEKÜLE ZUR VERWENDUNG ALS EMITTER**

ORGANIC MOLECULES FOR USE AS EMITTERS

MOLECULES ORGANIQUES DESTINEES A ETRE UTILISEES EN TANT QU'EMETTEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2016 DE 102016104929**
**01.09.2016 EP 16186804**

(43) Veröffentlichungstag der Anmeldung:
**23.01.2019 Patentblatt 2019/04**

(73) Patentinhaber: **cynora GmbH**
**76646 Bruchsal (DE)**

(72) Erfinder: **DANZ, Michael**
**76344 Eggenstein-Leopoldshafen (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2016/042070 US-A1- 2010 148 662**

- **KIM CW ET AL: "Thermally Stable Carbazole-Diimides as Hole Transport Materials for Organic Light-Emitting Devices", JOURNAL OF NANOSCIENCE AND NANOTECHNOLOGY, AMERICAN SCIENTIFIC PUBLISHERS, US, Bd. 12, Nr. 5, 1. Mai 2012 (2012-05-01), Seiten 4219-4223, XP009183046, ISSN: 1533-4880, DOI: 10.1166/JNN.2012.5891**
- **YU LIU ET.AL.: "New triphenylamine-based poly(amine-imide)s with carbazole-substituents for electrochromic applications", ORGANIC ELEMENTS, Bd. 15, 24. April 2014 (2014-04-24), Seiten 1422-1431, XP002767985,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 429 995 B1

## Beschreibung

Die Erfindung betrifft rein organische Moleküle ohne Metallzentren aufweisend eine Struktur der Formel A, sowie deren Verwendung als Emitter oder Host in optoelektronischen Bauelementen wie organischen lichtemittierenden Dioden (OLEDs).

## Einleitung

Optoelektronische Bauteile zeichnen sich dadurch aus, dass entweder elektrische Energie in Photonen umgewandelt wird (OLED, LEEC) oder der umgekehrte Prozess abläuft (OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Es muss jedoch ebenfalls in Betracht gezogen werden, dass in einer OLED 25 % Singulett-Excitonen und 75 % Triplett-Excitonen gebildet werden. Dies führt dazu, dass ein normaler Fluoreszenzemitter eine maximale interne Quantenausbeute von 25 % erreichen kann. Ebenso entsteht durch die gebildeten langlebigen, nicht emittierenden Triplettzustände das Problem einer stark abfallenden Effizienz bei höheren Strömen (sogenannter Effizienz roll-off).

Um diese Nachteile zu umgehen und möglichst alle Ladungsträger zu nutzen, kann das Konzept des Triplett-Harvesting genutzt werden. Hierzu müssen in der Regel teure Schweratom-Komplexe (z. B. Iridium oder Platin) verwendet werden (siehe hierzu z. B.: M. A. Baldo, D. F. O'Brian, M. E. Thompson, S. R. Forrest, Phys. Rev. B 1999, 60, 14422 und H. Yersin, Top. Curr. Chem. 2004, 241, 1).

Eine Alternative ist die Verwendung von Materialien, die thermisch aktivierte verzögerte Fluoreszenz (TADF) zeigen. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett- ($S_1$) und Triplett-Niveau ($T_1$). Dies kann beispielsweise durch Verwendung von Kupfer(I)-Komplexen, aber auch durch rein organische Materialien (siehe hierzu z. B.: H. Uoyama, K. Goushi, K. Shizu, H. Nomura, C. Adachi, Nature 2012, 492, 234) erreicht werden.

Die intensive Forschung in diesem Bereich zeigt, dass es weiterhin einen großen Bedarf an neuen Materialien gibt. Bekannte blaue TADF-Materialien zeigen oft hohe Exzitonenlebensdauern, welche die Effizienz und Lebensdauer von OLEDs negativ beeinflussen. Weiterhin ist eine einfache synthetische Zugänglichkeit relevant. Für die Synthese der Materialien sollten nicht nur entsprechende Synthesebausteine verfügbar sein, auch der Aufwand für die eigentliche Synthese des funktionellen Materials und dessen Aufreinigung sollten möglichst gering sein.

KIM, C.W. et. al. (Journal of Nanoscience and Nanotechnology , Bd. 12, Nr. 5, 2012, Seiten 4219-4223) beschreiben thermisch stabile Carbazol Diimide als Lochtransportmaterialien in OLEDs.

## Beschreibung der Erfindung

Die Erfindung betrifft in einem Aspekt die Bereitstellung von organischen Molekülen, insbesondere von Emittermolekülen, die eine Struktur der Formel A umfassen oder daraus bestehen:

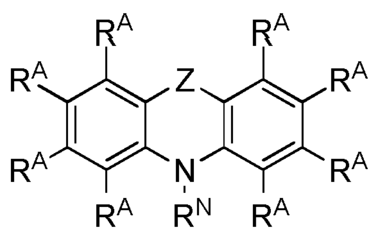

Formel A

Die in Formel A verwendeten Symbole haben folgende Bedeutung:

Z ist eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination der vorgenannten Gruppen, oder-CRR', -C=CRR', -C=NR, -NR-, -O-, SiRR'-, -S-, -S(O)-, $-S(O)_2-$, oder eine durch O unterbrochene substituierte oder unsubstituierte C1-C9-Alkylen-, C2-C8-Alkenylen-, C2-C8-Alkinylen- oder Arylen-Gruppe ist, oder Phenyl- oder substituierte Phenyleinheiten.

R und R' sind unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppen mit 2 bis 40 C-Atomen sowie verzweigte und cyclische Alkyl-, Alkenyl-, Alkinyl-, Alkoxy- und Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, das jeweils durch einen oder mehrere Reste $R^2$ substituiert sein können;
- Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen und Arylheteroarylaminogruppen mit 10 bis 40 aromatischen Ringatomen, welche durch einen oder mehrere Reste $R^2$ substituiert sein können;

wobei zwei oder mehrere der Substituenten R und R’ auch miteinander ein mono- oder polycyclisches, aliphatisches, aromatisches und/oder benzoannelliertes Ringsystem bilden können;

$R^N$ ist unabhängig voneinander ausgewählt aus der Gruppe bestehend aus:

- H, Deuterium;
- linearen Alkyl- und Alkoxy- und Thioalkoxygruppen mit 1 bis 40 C-Atomen und linearen Alkenyl- und Alkinylgruppen mit 2 bis 40 C-Atomen und verzweigten sowie cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können,
- aromatischen oder heteroaromatischen Ringsystemen mit 5 bis 60 aromatischen Ringatomen, die jeweils durch einen oder mehrere Reste $R^2$ substituiert sein können;
- Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können,

dabei können zwei oder mehrere dieser Substituenten $R^2$ auch miteinander ein mono- oder polycyclisches, aliphatisches, aromatisches und/oder benzoannelliertes Ringsystem bilden;

$R^A$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- und Thioalkoxygruppen mit 1 bis 40 C-Atomen und linearen Alkenyl- oder Alkinylgruppen mit 2 bis 40 C-Atomen und verzweigten sowie cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können;
- Aryloxy- oder Heteroaryloxygruppen mit 5 bis 60 aromatischen Ringatomen, die durch einen oder mehrere Reste $R^2$ substituiert sein können;
- einer Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann;
- einer Kombination der oben genannten Systeme und Gruppen; dabei können zwei oder mehrere der Substituenten $R^2$ auch miteinander ein mono- oder polycyclisches, aliphatisches, aromatisches und/oder benzoannelliertes Ringsystem bilden;
  und
- einer Gruppe gemäß Formel B, wobei mindestens eine Gruppe $R^A$ in Formel A eine Gruppe gemäß Formel B ist:

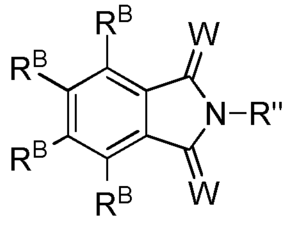

Formel B

In Formel B gilt:

W ist unabhängig voneinander ausgewählt aus der Gruppe bestehend aus $C(CN)_2$, $NR^2$, O und S;

R" ist bei jedem Auftreten gleich oder verschieden und ist wie $R^N$ definiert oder ist eine Einfachbindung oder eine organische Brücke B*, worüber eine zweite gleiche oder verschiedene Einheit der Formel B verbunden ist. An diese zweite Einheit der Formel B ist genau eine weitere (gleiche oder verschiedene) Einheit der Formel A gebunden, so dass eine dimere Struktur ausgebildet wird.

Die organische Brücke B* ist eine substituierte oder unsubstituierte Alkyl- (linear, verzweigt oder zyklisch), Heteroalkyl-, Aryl-, Heteroaryl- oder Alkenyl-Gruppen, insbesondere eine optional substituierte Phenyleinheit.

$R^2$ ist unabhängig voneinander ausgewählt aus der Gruppe bestehend aus:

- H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$, einer linearen Alkyl-, Alkoxy- oder Thioalkoxygruppe mit 1 bis 40 C-Atomen oder einer linearen Alkenyl- oder Alkinylgruppe mit 2 bis 40 C-Atomen oder einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, das jeweils durch einen oder mehrere Reste $R^3$ substituiert sein können;
- Aryloxy- oder Heteroaryloxygruppen mit 5 bis 60 aromatischen Ringatomen, die durch einen oder mehrere Reste $R^3$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^3$ substituiert sein können;

dabei können zwei oder mehrere der Substituenten $R^2$ auch miteinander ein mono- oder polycyclisches, aromatisches und/oder benzoannelliertes Ringsystem bilden.

$R^3$ ist bei jedem Auftreten gleich oder verschieden H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können. Zwei oder mehrere Substituenten $R^3$ können auch miteinander ein mono- oder polycyclisches, aliphatisches Ringsystem bilden.

$R^B$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, einer linearen Alkyl-, Alkoxy- oder Thioalkoxygruppe mit 1 bis 40 C-Atomen oder einer linearen Alkenyl- oder Alkinylgruppe mit 2 bis 40 C-Atomen oder einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können, oder Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;

wobei zwei oder mehrere dieser Substituenten R und $R^2$ auch miteinander ein mono- oder polycyclisches, aliphatisches, aromatisches und/oder benzoannelliertes Ringsystem bilden können.

Pro Einheit der Formel B, die in einem Molekül der Formel A vorhanden ist, stellt genau ein Rest $R^B$ eine Einfachbindung dar, über die die Gruppe gemäß Formel B an die Struktur gemäß Formel A bindet.

In einer Ausführungsform sind mindestens eine Gruppe $R^A$ und maximal zwei Gruppen $R^A$ in Formel A eine Gruppe gemäß Formel B.

In einer Ausführungsform ist Z gleich einer direkten Bindung, $R^N$ ist gleich unsubstituiertem oder substituiertem Phenyl, mit Substituenten wie Aryl, Alkyl und/oder CN, W ist gleich O und R" ist gleich alkyl- oder arylsubstituiertem Phenyl.

In einer Ausführungsform weist das organische Molekül eine Struktur der Formel C auf:

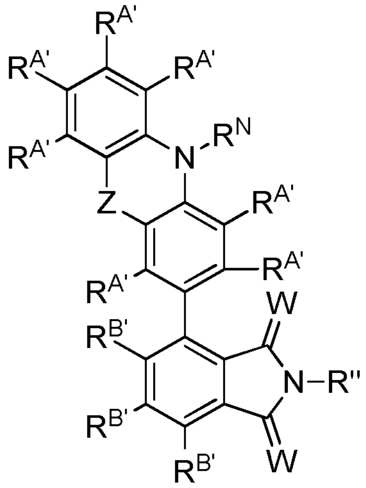

Formel C

In Formel C gilt:

$R^{B'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen oder linearen Alkenyl- oder Alkinylgruppen mit 2 bis 40 C-Atomen oder verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können, oder Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;

wobei zwei oder mehrere der Substituenten $R^2$ auch miteinander ein mono- oder polycyclisches, aliphatisches, aromatisches und/oder benzoannelliertes Ringsystem bilden können.

$R^{A'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen oder linearen Alkenyl- oder Alkinylgruppen mit 2 bis 40 C-Atomen oder verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder

CONR$^2$ ersetzt sein können und wobei ein oder mehrere H-Atome durch Deuterium, F, Cl, Br, I, CN, $CF_3$ oder $NO_2$ ersetzt sein können;

- aromatischen oder heteroaromatischen Ringsystemen mit 5 bis 60 aromatischen Ringatomen, das jeweils durch einen oder mehrere Reste R$^2$ substituiert sein können;
- Aryloxy- oder Heteroaryloxygruppen mit 5 bis 60 aromatischen Ringatomen, die durch einen oder mehrere Reste R$^2$ substituiert sein können;
- Diarylaminogruppe, Diheteroarylaminogruppe oder Arylheteroarylaminogruppe mit 10 bis 40 aromatischen Ringatomen, welche durch einen oder mehrere Reste R$^2$ substituiert sein können;
- einer Kombination der vorgenannten Systeme und Gruppen; dabei können zwei oder mehrere dieser Substituenten R$^2$ auch miteinander ein mono- oder polycyclisches, aliphatisches, aromatisches und/oder benzoannelliertes Ringsystem bilden;

  und
- einer Gruppe gemäß Formel B, wobei maximal ein R$^{A'}$ in der Formel C eine Gruppe gemäß Formel B ist.

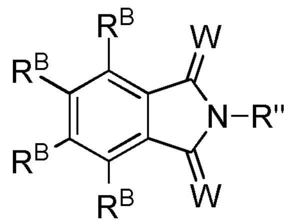

Formel B

Z, W, R", R$^N$, R$^B$, R, R', R$^2$, und R$^3$ sind wie bei Formel A und Formel B definiert.

In einer weiteren Ausführungsform weist das organische Molekül eine Struktur der Formel D auf:

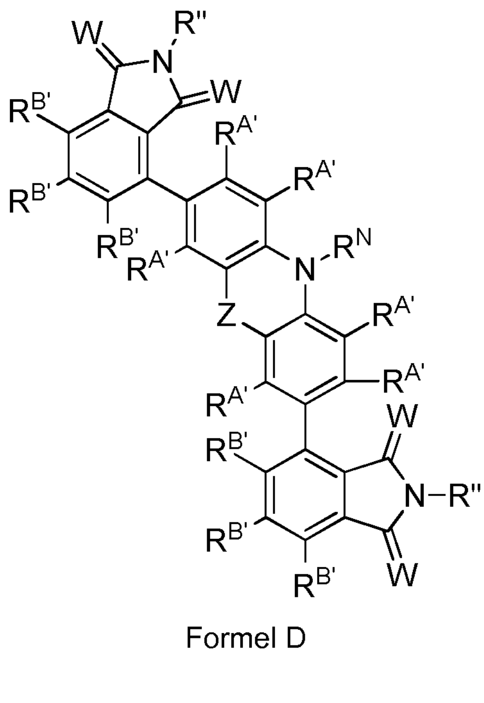

Formel D

In Formel D gilt:

R$^{B'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, COOR$^2$, $CO(NR^2)_2$, $Si(R^2)_3$, $B(OR^2)_2$, C(=O)R$^2$, $P(=O)(R^2)_2$, S(=O)R$^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppe mit 2 bis 40 C-Atomen und verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten R2 substituiert sein können, wobei eine oder mehrere nicht benachbarte $CH_2$-Gruppen durch R$^2$C=CR$^2$,

$C≡C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;

- aromatischen oder heteroaromatischen Ringsystemen mit 5 bis 60 aromatischen Ringatomen, das jeweils durch einen oder mehrere Reste $R^2$ substituiert sein können, oder eine Aryloxy- oder Heteroaryloxygruppe mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;

wobei zwei oder mehrere der Substituenten $R^2$ auch miteinander ein mono- oder polycyclisches, aliphatisches, aromatisches und/oder benzoannelliertes Ringsystem bilden können.

$R^{A'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppen mit 2 bis 40 C-Atomen und verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, $C≡C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können;
- Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können; und
- einer Kombination der vorgenannten Systeme und Gruppen; wobei zwei oder mehrere der Substituenten $R^2$ auch miteinander ein mono- oder polycyclisches, aliphatisches, aromatisches und/oder benzoannelliertes Ringsystem bilden können.

Z, W, R", $R^N$, R, R', $R^2$, und $R^3$ bei Formel D sind wie bei Formel A und Formel B definiert.

In einer weiteren Ausführungsform weist das organische Molekül eine Struktur der Formel E auf:

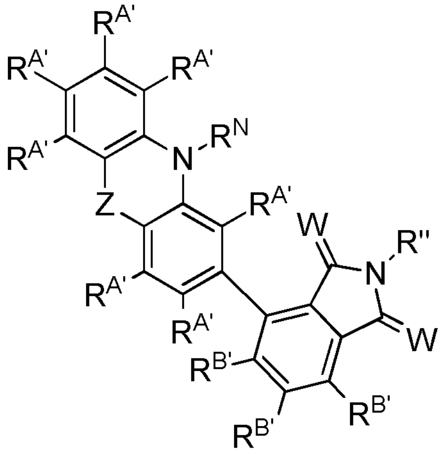

Formel E

In Formel E gilt:

$R^{B'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$,

$P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppen mit 2 bis 40 C-Atomen und verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten R2 substituiert sein können, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=P)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können, und Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;

wobei zwei oder mehrere der Substituenten $R^2$ auch miteinander ein mono- oder polycyclisches, aliphatisches, aromatisches und/oder benzoannelliertes Ringsystem bilden können.

$R^{A'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppen mit 2 bis 40 C-Atomen und verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, das jeweils durch einen oder mehrere Reste $R^2$ substituiert sein können;
- Aryloxy- oder Heteroaryloxygruppe mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;
- einer Kombination der vorgenannten Systeme und Gruppen; dabei können zwei oder mehrere der Substituenten $R^2$ auch miteinander ein mono- oder polycyclisches, aliphatisches, aromatisches und/oder benzoannelliertes Ringsystem bilden;
  und
- Gruppen gemäß der Formel B, wobei maximal ein $R^{A'}$ in der Formel E eine Gruppe gemäß Formel B ist.

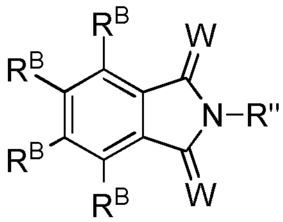

Formel B

Z, W, R'', $R^N$, $R^B$, R, R', $R^2$, und $R^3$ sind wie bei Formel A und Formel B definiert.

In einer weiteren Ausführungsform weist das organische Molekül eine Struktur der Formel F auf:

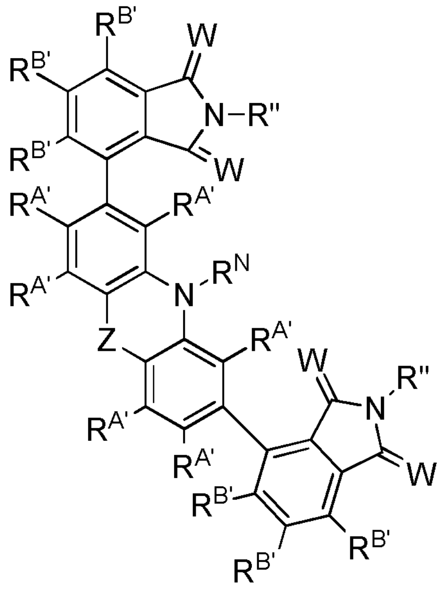

Formel F

In Formel F gilt:

$R^{B'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$, linearen Alkyl-, Alkoxy- oder Thioalkoxygruppen mit 1 bis 40 C-Atomen und linearen Alkenyl- oder Alkinylgruppen mit 2 bis 40 C-Atomen oder verzweigten oder cyclischen Alkyl-, Alkenyl-, Alkinyl-, Alkoxy- oder Thioalkoxygruppen mit 3 bis 40 C-Atomen, die jeweils mit einem oder mehreren Resten R2 substituiert sein können, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- aromatischen oder heteroaromatischen Ringsystemen mit 5 bis 60 aromatischen Ringatomen, die jeweils durch einen oder mehrere Reste $R^2$ substituiert sein können, und Aryloxy- oder Heteroaryloxygruppen mit 5 bis 60 aromatischen Ringatomen, die durch einen oder mehrere Reste $R^2$ substituiert sein können;
- Diarylaminogruppen, Diheteroarylaminogruppen oder Arylheteroarylaminogruppen mit 10 bis 40 aromatischen Ringatomen, welche durch einen oder mehrere Reste $R^2$ substituiert sein können;

wobei zwei oder mehrere der Substituenten $R^2$ auch miteinander ein mono- oder polycyclisches, aliphatisches, aromatisches und/oder benzoannelliertes Ringsystem bilden können.

$R^{A'}$ ist unabhängig voneinander ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- linearen Alkyl-, Alkoxy- oder Thioalkoxygruppen mit 1 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- linearen Alkenyl- oder Alkinylgruppe mit 2 bis 40 C-Atomen, die jeweils mit einem oder mehreren Resten $R^2$ substituiert sein können, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- verzweigten oder cyclischen Alkyl-, Alkenyl-, Alkinyl-, Alkoxy- oder Thioalkoxygruppen mit 3 bis 40 C-Atomen,

die jeweils mit einem oder mehreren Resten $R^2$ substituiert sein können, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;

- aromatischen oder heteroaromatischen Ringsystemen mit 5 bis 60 aromatischen Ringatomen, die jeweils mit einem oder mehreren Resten $R^2$ substituiert sein können;
- Aryloxy- oder Heteroaryloxygruppen mit 5 bis 60 aromatischen Ringatomen, die mit einem oder mehreren Resten $R^2$ substituiert sein können;

Diarylaminogruppen, Diheteroarylaminogruppen oder Arylheteroarylaminogruppen mit 10 bis 40 aromatischen Ringatomen, welche mit einem oder mehreren Resten $R^2$ substituiert sein können; und

- einer Kombination der vorgenannten Systeme und Gruppen; dabei können zwei oder mehrere der Substituenten R und $R^2$ auch miteinander ein mono- oder polycyclisches, aliphatisches, aromatisches und/oder benzoannelliertes Ringsystem bilden.

Z, W, R", $R^N$, R, R', $R^2$, und $R^3$ bei Formel F sind wie bei Formel A und Formel B definiert.

In einer weiteren Ausführungsform liegt das organische Molekül als Dimer vor und weist eine Struktur der Formel G auf

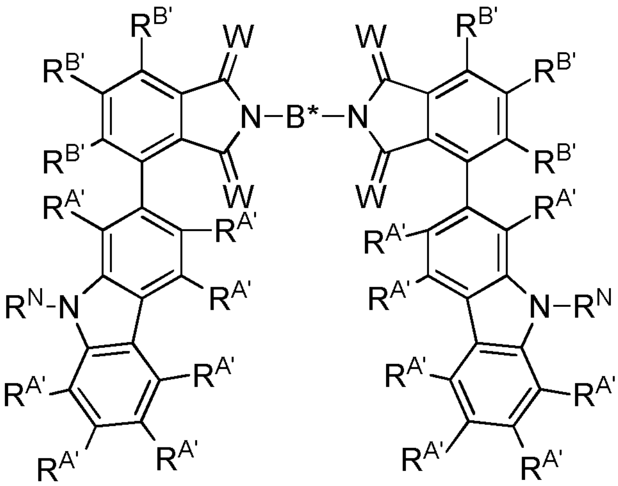

Formel G

wobei $R^{A'}$ und $R^{B'}$ wie bei Formel C definiert sind und im Übrigen die bei Formel A und Formel B angegebenen Definitionen gelten.

In einer weiteren Ausführungsform liegt das organische Molekül als Dimer vor und weist eine Struktur der Formel H auf

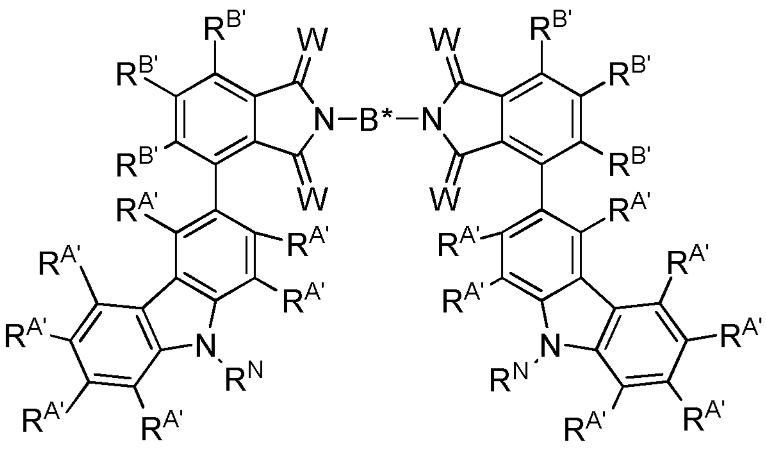

Formel H

wobei $R^{A'}$ und $R^{B'}$ wie bei Formel C definiert sind und im Übrigen die bei Formel A und Formel B angegebenen Definitionen gelten.

In einer Ausführungsform ist B* ausgewählt aus der Gruppe bestehend aus:

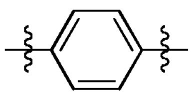

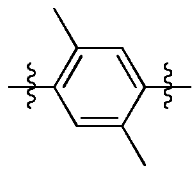

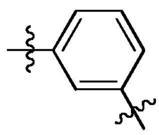

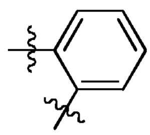

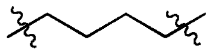

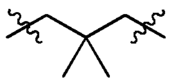

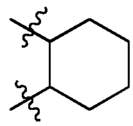

In speziellen Ausführungsformen weist das organische Molekül eine Struktur der Formel A, C, D, E oder F auf, in der W = O ist, R" eine Alkyleinheit oder eine Phenyleinheit ist, die optional alkyl-substituiert ist, und Z eine Einfachbindung ist.

In einer bevorzugten Ausführungsform weist das organische eine Struktur der Formel C oder D auf.

Ein weiterer Aspekt der Erfindung betrifft die Verwendung eines organischen Moleküls der hier beschriebenen Art als Emitter und/oder als Hostmaterial in einem optoelektronischen Bauelement.

Im Rahmen einer derartigen Verwendung ist das optoelektronische Bauelement insbesondere ausgewählt aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Gemäß zumindest einer Ausführungsform weist ein optoelektronische Bauelement zumindest zwei Elektroden auf, von denen zumindest eine durchsichtig ist und zwischen denen ein Schichtenstapel angeordnet ist, der zumindest eine organische Licht emittierende Schicht in Form einer organischen elektrolumineszierenden Schicht aufweist, die im Betrieb des optoelektronischen Bauelements Licht erzeugt. Das optoelektronische Bauelement kann insbesondere als organische lichtemittierende Diode (OLED) ausgebildet sein.

Eine derartiges optoelektronisches Bauelement weist in einer Ausführungsform auf:

- ein Substrat,
- eine Anode und
- eine Kathode,

wobei insbesondere die Anode oder die Kathode unmittelbar auf das Substrat aufgebracht sind, und

- mindestens eine lichtemittierende Schicht, die zwischen Anode und Kathode angeordnet ist und die das erfindungsgemäße organische Molekül aufweist.

In einem weiteren Aspekt betrifft die Erfindung ein Verfahren zur Herstellung eines optoelektronischen Bauelements. Dabei wird ein erfindungsgemäßes organisches Molekül der hier beschriebenen Art verwendet. In einer Ausführungsform umfasst das Herstellungsverfahren die Verarbeitung des erfindungsgemäßen organischen Moleküls mittels eines Vakuumverdampfungsverfahrens oder aus einer Lösung.

Die Erfindung betrifft in einem weiteren Aspekt ein Verfahren zur Herstellung eines optoelektronischen Bauelements, wobei ein erfindungsgemäßes organisches Molekül verwendet wird. In einer Ausführungsform dieses Verfahrens wird das erfindungsgemäße organische Molekül auf einen Träger aufgebracht, wobei das Aufbringen insbesondere nasschemisch, mittels kolloidaler Suspension oder mittels Sublimation erfolgt.

Erfindungsgemäß ist auch ein Verfahren zur Herstellung eines erfindungsgemäßen optoelektronischen Bauelements, bei dem mindestens eine Schicht des optoelektronischen Bauelements

- mit einem Sublimationsverfahren beschichtet werden,
- mit einem OVPD (Organic Vapour Phase Deposition) Verfahren beschichtet werden,
- mit einer Trägergassublimation beschichtet werden, und/oder
- aus Lösung oder mit einem Druckverfahren hergestellt werden.

In einem weiteren Aspekt betrifft der Erfindung eine Zusammensetzung aufweisend oder bestehend aus mindestens einem erfindungsgemäßen organischen Molekül, insbesondere als Emitter und/oder Host, und optional einer oder mehrerer von dem erfindungsgemäßen Molekül verschiedenen Emitter und/ oder Hosts, und optional einen oder mehreren Farbstoffen und/oder einem oder mehreren Lösungsmitteln.

Die hier verwendeten Begriffe "Aryl" und "aromatisch" sind als jegliche mono-, bi- oder polycyclische chemische Einheit zu verstehen. Beispielsweise kann ein Arylrest ein Phenylrest sein. Eine Arylgruppe kann auch wahlweise mit einer oder mehreren Substituenten substituiert sein, wie z. B. eine oder mehrere Substituenten ausgewählt aus der Gruppe von Aryl, Alkyl, Heteroryl, Heteroalkyl, Aralkyl, -O-aryl, -O-alkyl, -O-heteroaryl, -O-heteroalkyl, -O-aralkyl, -N-aryl, -N-alkyl, -N-heteroaryl, -N-heteroalkyl und/ oder -N-aralkyl, die jeweils weniger als 21 Kohlenstoffatome aufweisen, und/ oder Halogenid. Bevorzugt weist eine Aryleinheit nicht mehr als 20 Kohlenstoffatome auf, noch bevorzugter nicht mehr als 10 Kohlenstoffatome. Bevorzugt stellen die Arylsubstituenten substituierte oder unsubstituierte Phenylreste dar. Besonders bevorzugt sind Phenyl-, Tolyl-, Mesityl-, Anisyl-, Anilyl- und/ oder Anilinylreste.

Die hier verwendeten Begriffe "Heteroaryl" und "heteroaromatisch" sind als jegliche mono-, bi- oder polycyclische Einheit, die mindestens ein Heteroatom aufweisen zu verstehen, insbesondere ein bis drei Heteroatome pro aromatischem Ring. Solch ein Heteroatom kann beispielhaft aus Sauerstoff (N), Sauerstoff (O), Schwefel (S) oder Phosphor (P) gewählt sein. Beispielhafte heteroaromatische Verbindungen sind Pyrrol, Furan, Thiophen, Imidazol, Oxazol, Thiazol, Triazol, Pyrazol, Pyridin, Pyrazin, Pyrimidin etc. Enthält die heteroaromatische Gruppe mehrere Heteroatome, so sind alle Derivate, die sich lediglich in der relativen Position der Heteroatome unterscheiden, umfasst. Beispielhaft umfasst der Begriff Triazol Reste ausgewählt aus der Gruppe 1,2,3-Triazol und 1,2,4-Triazol. Eine Heteroarylgruppe kann auch wahlweise mit einer oder mehreren Substituenten, wie z. B. einer oder mehrerer Substituenten ausgewählt aus der Gruppe bestehend aus Aryl, Alkyl, Heteroryl, Heteroalkyl, Aralkyl, -O-aryl, -O-alkyl, -O-heteroaryl, -O-heteroalkyl, -O-aralkyl, -N-aryl, -N-alkyl, -N-heteroaryl, -N-heteroalkyl und -N-aralkyl, die jeweils weniger als 21 Kohlenstoffatome aufweisen, und Halogenid, substituiert sein. In einer Ausführungsform weist eine Heteroaryleinheit nicht mehr als 15 Kohlenstoffatome auf, insbesondere nicht mehr als 10 Kohlenstoffatome.

Der in hier verwendete Begriff "Alkyl" ist als unverzweigter oder verzweigter kettenförmiger oder zyklischer Alkylrest zu verstehen. Insbesondere enthalten die Alkylreste ein bis fünfzehn Kohlenstoffatome. Beispielhaft kann ein Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- oder Ähnliches darstellen. Eine Alkylgruppe kann auch wahlweise mit einem oder mehreren Substituenten, wie z. B. eine oder mehrere Substituenten ausgewählt aus der Gruppe von Aryl, Alkyl, Heteroryl, Heteroalkyl, Aralkyl, -O-aryl, -O-alkyl, -O-heteroaryl, -O-heteroalkyl, -O-aralkyl, -N-aryl, -N-alkyl, -N-heteroaryl, -N-heteroalkyl und/ oder -N-aralkyl, die jeweils weniger als 21 Kohlenstoffatome aufweisen, und/ oder Halogenid, substituiert sein. In einer Ausführungsform weist eine Alkyleinheit nicht mehr als 15 Kohlenstoffatome auf, insbesondere nicht mehr als 10 Kohlenstoffatome.

Der hier verwendete Begriff "Heteroalkyl" ist als unverzweigte oder verzweigte kettenförmiger oder zyklischer Alkylrest zu verstehen, der mindestens ein Heteroatom enthält. Beispielhaft kann ein Heteroalkylrest Methoxy, Trifluormethoxy, Ethoxy, n-Propoxy, i-Propoxy, n-Butoxy, i-Butoxy, s-Butoxy, t-Butoxy, 2-Methylbutoxy, Thiomethyl, Thioethyl oder Ähnliches darstellen. Eine Heteroalkylgruppe kann auch wahlweise mit einem oder mehreren Substituenten, wie z. B. eine oder mehrere Substituenten ausgewählt aus der Gruppe von Aryl, Alkyl, Heteroryl, Heteroalkyl, Aralkyl, -O-aryl, -O-alkyl, -O-heteroaryl, -O-heteroalkyl, -O-aralkyl, -N-aryl, -N-alkyl, -N-heteroaryl, -N-heteroalkyl und/ oder -N-aralkyl, die jeweils weniger als 21 Kohlenstoffatome aufweisen, und/oder Halogenid, substituiert sein. In einer Ausführungsform weist eine Heteroalkyleinheit nicht mehr als 15 Kohlenstoffatome auf, insbesondere nicht mehr als 10 Kohlenstoffatome.

Der hier verwendeten Begriff "Halogenid" bezieht sich auf Fluorid, Chlorid, Bromid und Iodid.

Der hier verwendete Begriff "Aralkyl" ist als mit einem Arylrest substituierte Alkylrest zu verstehen. Eine Aralkylgruppe kann auch wahlweise mit einem oder mehreren Substituenten, wie z. B. eine oder mehrere Substituenten ausgewählt aus der Gruppe bestehend aus Aryl, Alkyl, Heteroryl, Heteroalkyl, Aralkyl, -O-aryl, -O-alkyl, -O-heteroaryl, -O-heteroalkyl, -O-aralkyl, -N-aryl, -N-alkyl, -N-heteroaryl, -N-heteroalkyl und -N-aralkyl, die jeweils weniger als 21 Kohlenstoffatome aufweisen, und/oder Halogenid, substituiert sein. In einer Ausführungsform weist eine Heteroalkyleinheit nicht mehr als 15 Kohlenstoffatome auf, insbesondere nicht mehr als 10 Kohlenstoffatome.

Als Emitter sind im Sinne der Erfindung sind Verbindungen zu verstehen, die eine Emission im sichtbaren Bereich von 380 nm bis 800 nm aufweisen.

In einer Ausführungsform sind unter Emittern Verbindungen zu verstehen, die ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm aufweisen.

Die erfindungsgemäßen Verbindungen zeichnen sich insbesondere durch eine wohldefinierte Emission mit nur einem Emissionspeak aus.

Der Anteil des erfindungsgemäßen organischen Moleküls als Emitter beträgt in einer weiteren Ausführungsform in einer lichtemittierenden Schicht in optischen Licht emittierenden Bauelementen, insbesondere in OLEDs, zwischen 5 % und 80 %. In einer Ausführungsform des erfindungsgemäßen optoelektronischen Bauelements ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

In einer Ausführungsform stellt das organische Molekül der vorliegenden Erfindung einen Emitter dar, der thermisch aktivierte verzögerte Fluoreszenz (TADF) aufweist.

In einer weiteren bevorzugten Ausführungsform weist das organische Molekül der vorliegenden Erfindung eine Energiedifferenz zwischen dem ersten angeregten Singulett-Zustand und dem ersten angeregten Triplett-Zustand ($\Delta E(S_1-T_1)$) von nicht höher als 5000 $cm^{-1}$, insbesondere nicht höher als 3000 $cm^{-1}$, oder nicht höher als 1500 $cm^{-1}$ oder 1000 $cm^{-1}$ auf.

Die Bestimmung des $\Delta E(S_1-T_1)$-Wertes kann sowohl durch quantenmechanische Berechnungen mittels im Stand der Technik bekannten Computerprogrammen durchgeführt werden (z. B. mittels Turbomole-Programmen unter Ausführung von TD-DFT- und unter Berücksichtigung von CC2-Rechnungen) oder - wie weiter unten erläutert wird - experimentell bestimmt werden.

Die Energiedifferenz $\Delta E(S_1-T_1)$ lässt sich näherungsweise quantenmechanisch durch das mit dem Faktor 2 multiplizierte sogenannte Austauschintegral beschreiben. Dessen Wert hängt direkt ab von der Überlappung der Molekülorbitale. Diese Molekülorbitale sind über unterschiedliche Raumbereiche verteilt (teil-delokalisiert über $\pi$- bzw. $\pi^*$-Molekülorbitale). Das heißt, ein elektronischer Übergang zwischen den verschiedenen Molekülorbitalen repräsentiert einen sogenannten Charge-Transfer (CT)-Übergang. Je geringer die Überlappung der oben beschriebenen Molekülorbitale ist, desto ausgeprägter ist der elektronische Charge-Transfer Charakter. Das ist dann mit einer Abnahme des Austausch-Integrals und somit einer Abnahme der Energiedifferenz $\Delta E(S_1-T_1)$ verbunden.

Die Bestimmung des $\Delta E(S_1-T_1)$-Wertes kann experimentell folgendermaßen erfolgen:
Für ein vorgegebenes organisches Molekül lässt sich der Energieabstand $\Delta E(S_1-T_1) = \Delta E$ unter Verwendung der oben angegebenen Gleichung (1) einfach bestimmen. Eine Umformung ergibt:

$$\ln\{Int(S_1 \rightarrow S_0)/Int(T_1 \rightarrow S_0)\} = \ln\{k(S_1)/k(T_1)\} - (\Delta E/k_B)(1/T) \quad (3)$$

Für die Messung der Intensitäten $Int(S_1 \rightarrow S_0)$ und $Int(T_1 \rightarrow S_0)$ kann jedes handelsübliche Spektralphotometer verwendet werden. Eine graphische Auftragung der bei verschiedenen Temperaturen gemessenen (logarithmierten) Intensitätsverhältnisse $\ln\{Int(S_1 \rightarrow S_0)/Int(T_1 \rightarrow S_0)\}$ gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei $Int(S_1 \rightarrow S_0)$ bzw. $Int(T_1 \rightarrow S_0)$ die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol $L^{-1}$) oder an dünnen Filmen aus den entsprechenden Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyl-THF, THF (Tetrahydrofuran) oder aliphatische Kohlenwasserstoffe. Verwendet man als Probe einen Film, so eignet sich die Verwendung einer Matrix mit einer deutlich größeren Singulettsowie Triplett-Energie als die der organischen Emittermoleküle, z. B. PMMA (Polymethylmethacrylat). Dieser Film kann aus Lösung aufgebracht werden.

Die Geradensteigung beträgt $-\Delta E/k_B$. Mit $k_B$ = 1,380 $10^{-23}$ $JK^{-1}$ = 0,695 $cm^{-1}$ $K^{-1}$ lässt sich der Energieabstand direkt bestimmen.

Eine äquivalente Betrachtungsweise zeigt, dass mittels der Messung der Temperaturabhängigkeit der Emissionsabklingzeit der $\Delta E(S_1\text{-}T_1)$-Wert auch bestimmt werden kann.

Eine einfache, näherungsweise Abschätzung des $\Delta E(S_1\text{-}T_1)$-Wertes kann auch dadurch vorgenommen werden, dass bei tiefer Temperatur (z. B. 77 K oder 4,2 K unter Verwendung eines Kryostaten) die Fluoreszenz- und Phosphoreszenz-Spektren registriert werden. Der $\Delta E(S_1\text{-}T_1)$-Wert entspricht dann in Näherung der Energiedifferenz zwischen den hochenergetischen Anstiegsflanken der Fluoreszenz- bzw. Phosphoreszenz-Bande.

Je ausgeprägter der CT-Charakter eines organischen Moleküls ist, desto stärker verändern sich die elektronischen Übergangsenergien als Funktion der Lösungsmittelpolarität. So gibt bereits eine ausgeprägte Polaritätsabhängigkeit der Emissionsenergien einen Hinweis auf das Vorliegen kleiner $\Delta E(S_1\text{-}T_1)$-Werte.

Als Host sind Materialien zu verstehen, welche Teil der lichtemittierenden Schicht sind und in welche die emittierenden Moleküle eingebettet sind. Die lichtemittierende Schicht kann eine oder mehrere Hostkomponenten enthalten. Hostmaterialien müssen eine effiziente Energieniveau-Nivellierung ermöglichen, um die Energieniveaus der verwendeten Elektroden in Kombination mit den Transportmaterialien stufenweise an die Energieniveaus der Emittermoleküle anzupassen. Weiterhin sollten das verwendete Hostmaterial oder die verwendeten Hostmaterialien idealerweise einen ausgeglichenen Ladungstransport (eine Ladungsträgerbalance von positiven und negativen Ladungsträgern) gewährleisten, sowie Triplett-Triplett-Annihilation und konzentrationsbedingte Selbstauslöschung der Emitter minimieren. Bevorzugt weisen Hostmaterialien ausreichend hohe Bandlücken auf, um einen effektiven Elektronen- oder Energietransfer zu den Emitterkomponenten zu ermöglichen. Beispielhafte gängige Hostmaterialien sind CBP, mCP, TPBi und TCTA, die in der Lage sind, entweder bevorzugt Löcher oder bevorzugt Elektronen zu transportieren, und ambipolare Hostmaterialien, die sowohl Löcher als auch Elektronen transportieren.

Das organische Molekül kann über beliebige Wege synthetisiert werden. Beispielhafte Syntheserouten sind in den Ausführungsbeispielen beschrieben.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und

(b) mindestens einem (d. h. einem, zwei oder mehreren) Emitter- und/oder Hostmaterialien, die von dem erfindungsgemäßen organischen Molekül verschiedenen ist bzw. sind und

(c) optional mindestens einem Farbstoff und/ oder mindestens einem organischen Lösungsmittel.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das jeweilige des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt eine weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)

2. Anode

3. Lochinjektionsschicht (hole injection layer, HIL)

4. Lochtransportschicht (hole transport layer, HTL)

5. Elektronenblockierschicht (electron blocking layer, EBL)

6. Emitterschicht (emitting layer, EML)

7. Lochblockierschicht (hole blocking layer, HBL)

8. Elektronenleitschicht (electron transport layer, ETL)

9. Elektroneninjektionsschicht (electron injection layer, EIL)

10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)

2. Kathode

3. Elektroneninjektionsschicht (electron injection layer, EIL)

4. Elektronenleitschicht (electron transport layer, ETL)

5. Lochblockierschicht (hole blocking layer, HBL)

6. Emissionsschicht bzw. Emitterschicht (emitting layer, EML)

7. Elektronenblockierschicht (electron blocking layer, EBL)

8. Lochtransportschicht (hole transport layer, HTL)

9. Lochinjektionsschicht (hole injection layer, HIL)

10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einer p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein.

Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen.

Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oderSpiro-NPD (N,N'-diphenyl-N,N'-Bis-(1-naphthyl)-9,9'-spirobifluorene-2,7-diamin) dienen. Beispielhaft ist die Schichtdicke 10-80 nm. Des Weiteren können kleine Moleküle verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N,N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10-100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid,

Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

## Beispiele

### Allgemeine Vorschriften

### Berechnungen nach der Dichtefunktionaltheorie

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D. , J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J. Chem. Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F. ; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, eine Entwicklung der Universität Karlsruhe und Forschungszentrum Karlsruhe GmbH, 1989-2007, TURBOMOLE GmbH, seit 2007; http://www. turbomole.com) durchgeführt.

Die in den Figuren dargestellten Grenzorbitale wurden mit dem B3LYP-Funktional für die optimierten Grundzustandsgeometrien berechnet.

### Photophysikalische Messungen

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.

***Probenvorbereitung, Film: Spin-Coating*** (Gerät: Spin150, SPS euro.)

Die Probenkonzentration entsprach 10mg/ml, angesetzt in Toluol oder Chlorbenzol.

Programm: 1) 3 s bei 400 U/min; 2) 20 Sek. bei 1000 U/min bei 1000 Upm/ s. 3) 10 s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen: UV-VIS-Spektren wurden auf einem Gerät der Firma Thermo Scientific, Modell Evolution 201 aufgenommen. (Siehe Probenvorbereitung: Lösungen)

Film: UV-VIS-Spektren wurden auf einem Gerät der Firma Thermo Scientific, Modell Evolution 201 aufgenommen. (Siehe Probenvorbereitung, Film: Spin-Coating)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen: NanoLED 370 (Wellenlänge: 371 nm, Pulsdauer: 1.1 ns), NanoLED 290 (Wellenlänge: 294 nm, Pulsdauer: <1 ns), SpectraLED 310 (Wellenlänge: 314nm), SpectraLED 355 (Wellenlänge: 355nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: Gemessenen Größe.

***Quanteneffizienzbestimmung***

Die Messung der Photolumineszenzquantenausbeute (PLQY) erfolgte mittels eines *Absolute PL Quantum Yield Measurement* C9920-03G-Systems der Firma *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0

Für G9920-OXG (PMA-12). Das Emissionsmaximum wird in nm, die Quantenausbeute Φ in % und die CIE-Farbkoordinaten als x,y-Werte angegeben.

Die PLQY wurde für Polymerfilme, Lösungen und Pulverproben nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorptionsmaximum des organischen Moleküls bestimmt und mit diesem angeregt.
3) Durchführung der Probenmessung: Es wurde von entgasten Lösungen und Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Herstellung und Charakterisierung von organischen Elektrolumineszenzvorrichtungen aus der Gasphase**

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

Die Bedeutung der in Figur 1 gezeigten Schichten ist wie folgt:

1. Als Trägermaterial kann Glas oder jedes andere geeignete feste oder flexible durchsichtige Material verwendet werden.
2. Anode = ITO = Indium-Zinn-Oxid
3. HIL = hole injection layer = Lochinjektionschicht. Hierfür kann beispielsweise das kommerziell erhältliche PEDOT:PSS verwendet werden. Typische Schichtdicke: 20 nm bis 80 nm.
Kleine Moleküle können auch verwendet werden (z. B. Kupfer-Phthalocyanin (CuPc z. B. 10 nm dick)) oder Metalloxide z.B. MoO3, V2O5, etc.
HIL kann auch als Anode angesehen werden.
4. HTL = hole transport layer = Lochtransportschicht. Diese Schicht ist wie hinten ausgeführt, optional, kann jedoch zur Verbesserung der Bauteileigenschaften verwendet werden. Hierfür kann z. B. [alpha]-NPD (N,N'-Di(1-naphthyl)-N,N'-diphenyl-(1,1'-biphenyl)-4,4'-diamin), TAPC (4,4'-Cyclohexyliden-bis[N,N-bis(4-methylphenyl)benzenamine]), TCTA (Tris(4-carbazoyl-9-ylphenyl)amin), mCP (1,3-Bis(N-carbazolyl)benzen) oder TrisPCz (9,9'-Diphenyl-6-(9-phenyl-9H-carbazol-3-yl)-9H,9'H-3,3'-bicarbazol) in einer Dicke von z. B. 20 nm eingesetzt werden.
5. EBL = Die Elektronenblockierschicht (optional) soll dafür sorgen, dass der Elektronentransport zur Anode unterbunden wird, da die Ladungsträger in die Emitter-Schicht gehalten werden und die Lochtransportschicht nicht durch Elektronen degradiert wird (Dicke z. B. 30 nm). Schicht 4 und 5 können ein einziges Material sein wenn die HTL sowohl gute Eigenschaften als Lochtransport- und Elektronenblockierschicht aufweist.
6. EML = Die Emitter-Schicht enthält oder besteht aus dem erfindungsgemäßen organischen Molekül. Dieses kann für die sublimierbaren erfindungsgemäßen Materialien durch Sublimation aufgetragen werden. Die Schichtdicke kann z. B. zwischen 10 nm und 200 nm liegen. Für im Grünen oder im Roten emittierende Emittermaterialien eignen sich die gängigen Matrixmaterialien wie CBP (4,4'-Bis-(N-carbazolyl)- biphenyl). Für im Blauen emittierende Emittermaterialien können UGH-Matrixmaterialien (siehe z. B. M.E. Thompson et al., Chem. Mater. 2004, 16, 4743) oder andere sogenannten Wide-Gap-Matrixmaterialien eingesetzt werden.
7. HBL= Hole Blocking Layer =Die Lochblockierschicht soll die Ladungsträger in die Emitterschicht halten (optional). Diese Schicht kann z. B. 10 nm dick sein. Als Material eignet sich z. B. BCP (4,7-Diphenyl-2,9- dimethyl-phenanthrolin = Bathocuproin) oder TSPO1 (Diphenyl-4-triphenylsilylphenyl-phosphinoxid).
8. ETL = Electron Transport Layer = Elektronentransportschicht. Zum Beispiel kann das aufdampfbare Alq3 (Aluminium-tris(8-hydroxychinolin)) oder TPBi (1,3,5-Tris(1-phenyl-1H-benzimidazol-2-yl)benzen) verwendet werden. Dicke ist z. B. 25 nm. Schichte 7 und 8 können ein einziges Material sein wenn die ETL sowohl gute Eigenschaften als Elektronentransport- und Lochblockierschicht aufweist.
9. Kathode. Diese Schicht wird in der Regel aufgedampft und ist z.B LiF, Ca, Mg, Ba. Gegebenenfalls können die ETL- und die CsF-Schicht entfallen. Die Schichtdicke beträgt zwischen 0.5 und 30 nm.
10. Al oder anderen stabile Metalle (Cu, Ag, etc.) werden aufgedampft um die reaktive Kathode zu beschützen. Al kann auch mit der unteren Schicht reagieren (z.B. LiF/Al) (Xie, Z. T., Zhang, W. H., Ding, B. F., Gao, X. D., You, Y. T., Sun, Z. Y., ... Hou, X. Y. (2009). Interfacial reactions at Al/LiF and LiF/Al. Applied Physics Letters, 94(6), 063302. doi:10.1063/1.3077167). Typische Schichtdicken betragen 100 nm.

Die am Device anliegende Spannung beträgt z. B. 2,5 V bis 15 V.

**Beispiele**

Synthese Vorstufe:

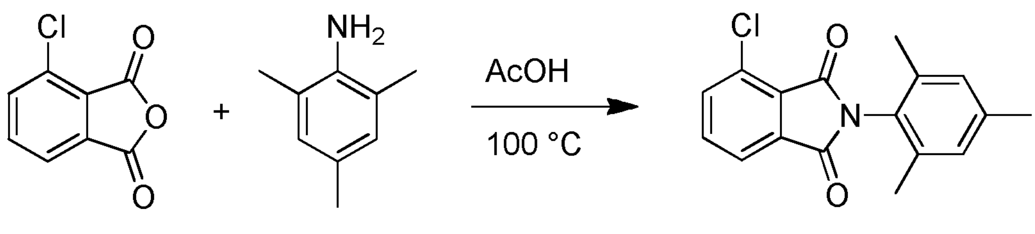


**A1**

In einem 100 mL Rundkolben mit Rückflusskühler werden 3-Chlorphtalsäureanhydrid (4.56 g, 25 mmol) in 50 mL Eisessig suspendiert. Nach Zugabe von 2,4,6-Trimethylanilin (3,72 g, 27,5 mmol) wird für 3 h bei 100 °C gerührt. Nach Abkühlen wird die Essigsäure größtenteils am Rotationverdampfer entfernt. Der Rückstand wird in $CH_2Cl_2$ aufgenommen und über Kieselgel filtriert (Eluent: $CH_2Cl_2$). Das Lösungsmittel wird am Rotationsverdampfer entfernt. Nach Trocknen am Vakuum wird das Produkt A1 (6,01 g, 20,0 mmol, 80%) als weißer Feststoff erhalten.
$^1$H-NMR (500 MHz, $CDCl_3$): $\delta$ = 7.89-7.85 (m, 1H), 7.74 - 7.68 (m, 2H), 7.00 (s, 2H), 2.33 (s, 3H), 2.13 (s, 6H).

Die weiteren Vorstufen werden analog erhalten, wobei statt 2,4,6-Trimethylanilin andere Amine eingesetzt werden. Beispielhaft wird A2 durch Reaktion von 3-Chlorphtalsäureanhydrid mit 2-Phenylanilin und A3 durch Reaktion

von 3-Chlorphtalsäureanhydrid mit Anilin erhalten.

**Beispiel 1**

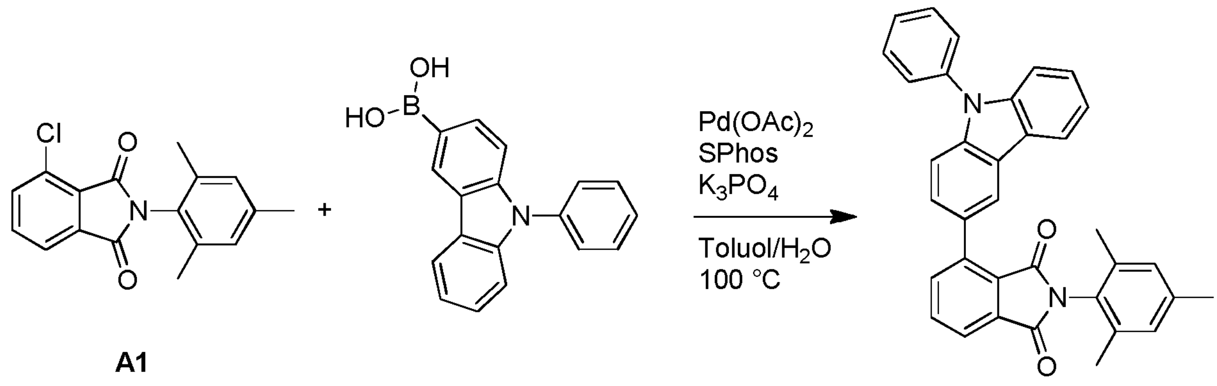


A1

1

In einem 100 mL Zweihalskolben mit Rückflusskühler werden unter Schutzgas Verbindung **A1** (1,5 g, 5 mmol), (9-Phenyl-9H-carbazol-3-yl)boronsäure (2,15 g, 7,5 mmol), $Pd(OAc)_2$ (34 mg, 0,15 mmol), SPhos (123 mg, 0,3 mmol; CAS-Nummer 657408-07-6) und $K_3PO_4$ (2,12 g, 10 mmol) vorgelegt und 3x evakuiert und wieder mit Stickstoff belüftet. Nach Zugabe von 15 mL Toluol (entgast) und 2 mL Wasser (entgast) wird 5 min Stickstoff durch die Reaktionslösung geleitet. Und anschließend 1 h bei 100 °C gerührt. Nach abkühlen wird die Reaktionslösung über wenig Kieselgel filtriert (Eluent: $CH_2Cl_2$). Das Lösungsmittel wird am Rotationsverdampfer entfernt. Umkristallisation aus EtOH/$CHCl_3$ liefert Verbindung **1** als gelben Feststoff (1,1 g, 2,17 mmol, 43%).

$^1$H-NMR (500 MHz, $CDCl_3$): $\delta$ = 8.43 (d, J = 1.6 Hz, 1H), 8.17 (dt, J = 7.8, 1.0 Hz, 1H), 7.97 (dd, J = 7.2, 1.2 Hz, 1H), 7.90 (dd, J = 7.8, 1.2 Hz, 1H), 7.87 - 7.82 (m, 1H), 7.70 (dd, J = 8.5, 1.8 Hz, 1H), 7.64 - 7.56 (m, 4H), 7.51 - 7.45 (m, 2H), 7.45 - 7.40 (m, 2H), 7.30 (ddd, J = 8.0, 5.1, 3.0 Hz, 1H), 6.98 (s, 2H), 2.31 (s, 3H), 2.16 (s, 6H).

Für 1 wurde eine Photolumineszenzquantenausbeute (PLQY) von 57 % (Film 10 % in PMMA) und ein Emissionsmaximum von 482 nm bestimmt. Das Emissionsspektrum ist in Figur 2 dargestellt.

**Beispiel 2**

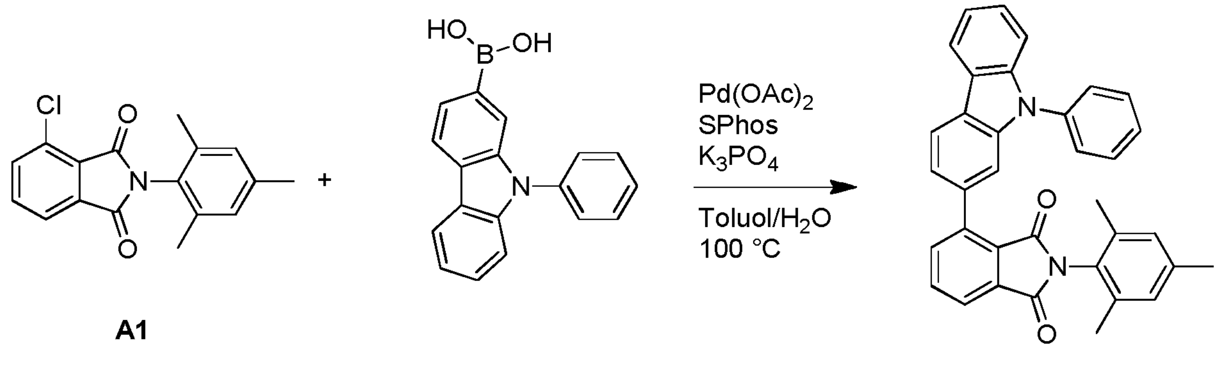


A1

2

In einem 100 mL Zweihalskolben mit Rückflusskühler werden unter Schutzgas Verbindung **A1** (899 mg, 3 mmol), (9-Phenyl-9H-carbazol-2-yl)boronsäure (1,12 g, 3,9 mmol), $Pd(OAc)_2$ (20 mg, 90 $\mu$mol), SPhos (74 mg, 180 $\mu$mol) und $K_3PO_4$ (1,27 g, 6 mmol) vorgelegt und 3x evakuiert und wieder mit Stickstoff belüftet. Nach Zugabe von 10 mL Toluol (entgast) und 1 mL Wasser (entgast) wird 5 min Stickstoff durch die Reaktionslösung geleitet. Und anschließend

1 h bei 100 °C gerührt. Nach abkühlen wird die Reaktionslösung über wenig Kieselgel filtriert (Eluent: $CH_2Cl_2$). Das Lösungsmittel wird am Rotationsverdampfer entfernt. Umkristallisation aus EtOH/$CHCl_3$ liefert Verbindung **2** als gelben Feststoff (1,18 g, 2,33 mmol, 78%).

$^1$H-NMR (500 MHz, $CDCl_3$): δ = 8.23 (d, J = 8.1 Hz, 1H), 8.19 (dd, J = 7.8, 1.0 Hz, 1H), 7.94 (dd, J = 6.9, 1.5 Hz, 1H), 7.82 (dd, J = 7.8, 1.5 Hz, 1H), 7.79 (dd, J = 7.8, 6.9 Hz, 1H), 7.72 (d, J = 1.3 Hz, 1H), 7.69-7.65 (m, 2H), 7.58 (dd, J = 8.7, 7.1 Hz, 2H), 7.55 (dd, J = 8.1, 1.5 Hz, 1H), 7.49 - 7.42 (m, 3H), 7.32 (ddd, J = 8.0, 6.7, 1.3 Hz, 1H), 7.01 (s, 2H), 2.34 (s, 3H), 2.14 (s, 6H).

Für 2 (Film 10 % in PMMA) wurde ein Emissionsmaximum von 484 nm bestimmt. Das Emissionsspektrum ist in Figur 3 dargestellt.

Beispiel 3:

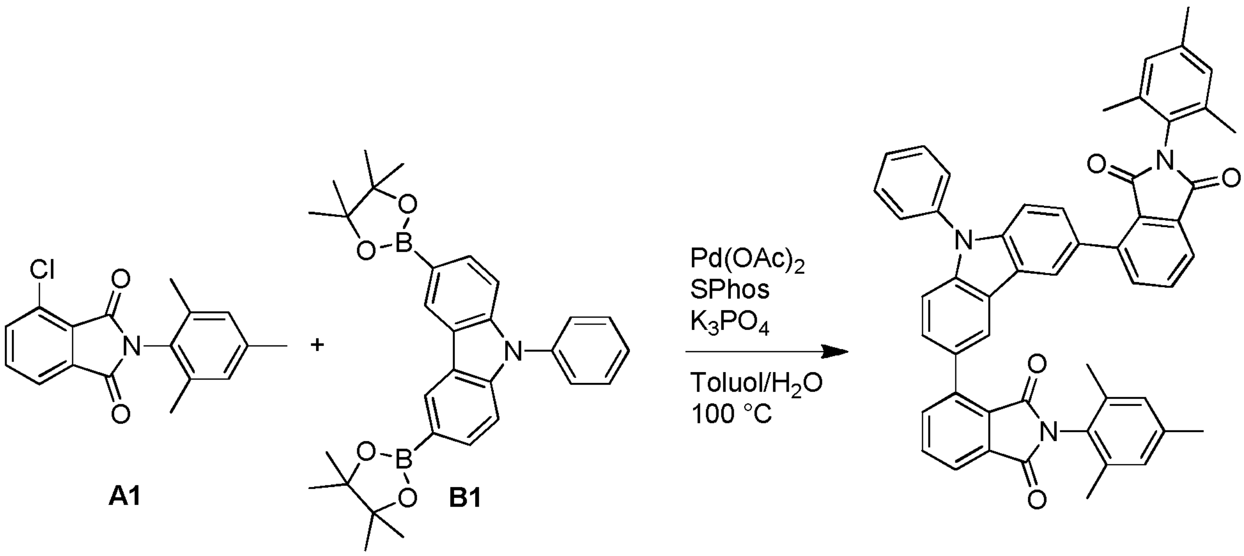


**A1** + **B1** → **3**

3 Äquivalente **A1** werden mit einem Äquivalent **B1** analog den Reaktionsbedingungen der Beispiele 1 und 2 umgesetzt. Die Verbindung 3 wird als gelber Feststoff erhalten (Ausbeute 76 %).

$^1$H NMR (500 MHz, Chloroform-d) δ 8.44 (d, *J* = 1.7 Hz, 2H), 7.96 (dd, *J* = 7.2, 1.1 Hz, 2H), 7.90 (dd, *J* = 7.8, 1.2 Hz, 2H), 7.87 - 7.81 (m, 2H), 7.69 (dd, *J* = 8.5, 1.8 Hz, 2H), 7.64 - 7.57 (m, 4H), 7.52-7.47 (m, 1H), 7.46 (d, *J* = 8.6 Hz, 2H), 6.96 (s, 4H), 2.30 (s, 6H), 2.13 (s, 12H).

Für **3** wurde eine Photolumineszenzquantenausbeute (PLQY) von 67 % (Film 10 % in PMMA) und ein Emissionsmaximum von 485 nm bestimmt. Das Emissionsspektrum ist in Figur 4 dargestellt.

**Beispiel 4:**

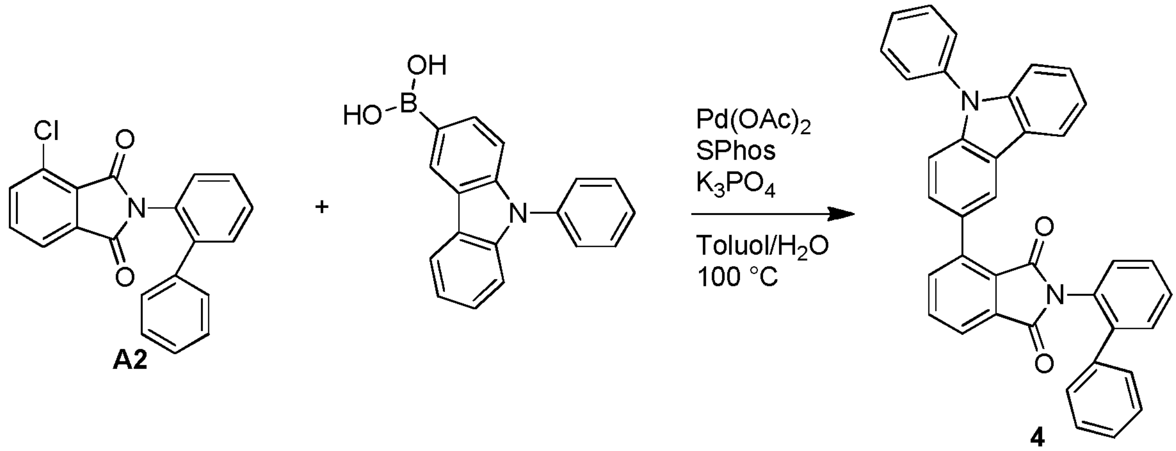


A2

4

Vorstufe **A2** wird analog zu **A1** durch Reaktion von 3-Chlorphtalsäureanhydrid mit 2-Phenylanilin hergestellt. Anschließend erfolgt eine Umsetzung mit (9-Phenyl-9H-carbazol-2-yl)boronsäure analog Beispiel 2. Die Verbindung **4** wird als gelber Feststoff erhalten (Ausbeute 35 %).

$^1$H NMR (500 MHz, Chloroform-d) δ 8.21 (d, *J* = 1.8, Hz, 1H), 8.14 (dt, *J* = 7.9, 1.0 Hz, 1H), 7.82 (dd, *J* = 7.1, 1.4 Hz, 1H), 7.80 - 7.77 (m, 1H), 7.77-7.73 (m, 1H), 7.65 - 7.60 (m, 2H), 7.60 - 7.56 (m, 2H), 7.54 - 7.45 (m, 5H), 7.45 - 7.39 (m, 3H), 7.37 - 7.26 (m, 7H).

Für **4** wurde eine Photolumineszenzquantenausbeute (PLQY) von 66 % (Film 10 % in PMMA) und ein Emissionsmaximum von 480 nm bestimmt. Das Emissionsspektrum ist in Figur 5 dargestellt.

**Beispiel 5:**

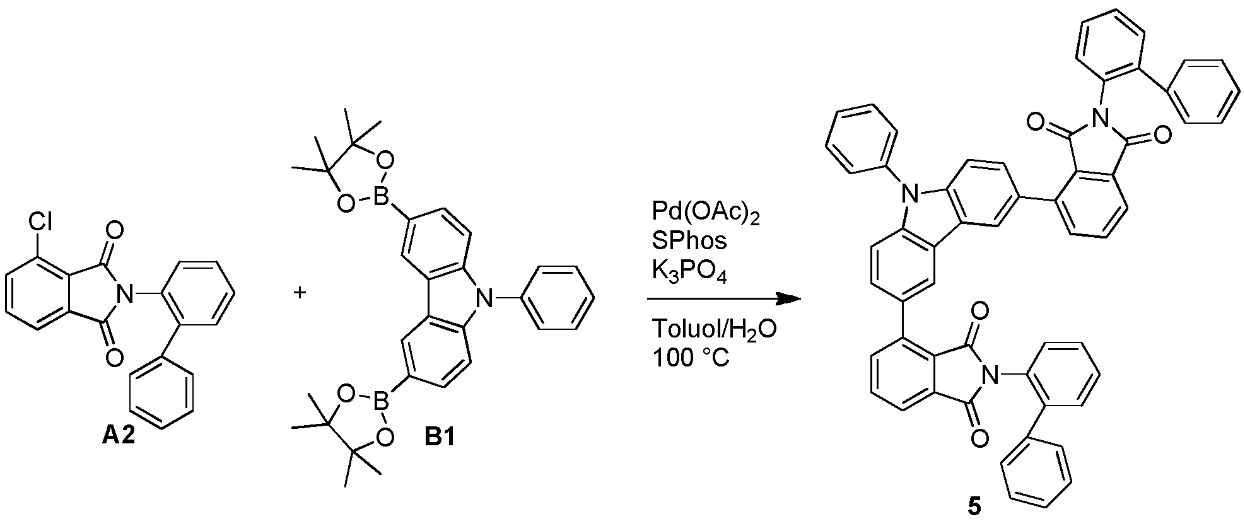


A2 B1

5

3 Äquivalente **A2** werden mit einem Äquivalent **B1** analog den Reaktionsbedingungen der Beispiele 1 und 2 umgesetzt. Die Verbindung **5** wird als gelber Feststoff erhalten (Ausbeute 73 %).

$^1$H NMR (500 MHz, Chloroform-*d*) δ 8.22 (dd, *J* = 12.0, 1.7 Hz, 2H), 7.84-7.79 (m, 4H), 7.79 - 7.74 (m, 2H), 7.65-7.60 (m, 2H), 7.60 - 7.57 (m, 2H), 7.53 - 7.44 (m, 9H), 7.41 (d, *J* = 8.5 Hz, 2H), 7.37 - 7.33 (m, 2H), 7.33-7.29 (m, 4H), 7.29 - 7.21 (m, 6H).

Für **5** wurde eine Photolumineszenzquantenausbeute (PLQY) von 65 % (Film 10 % in PMMA) und ein Emissionsmaximum von 487 nm bestimmt. Das Emissionsspektrum ist in Figur 6 dargestellt.

**Beispiel 6:**

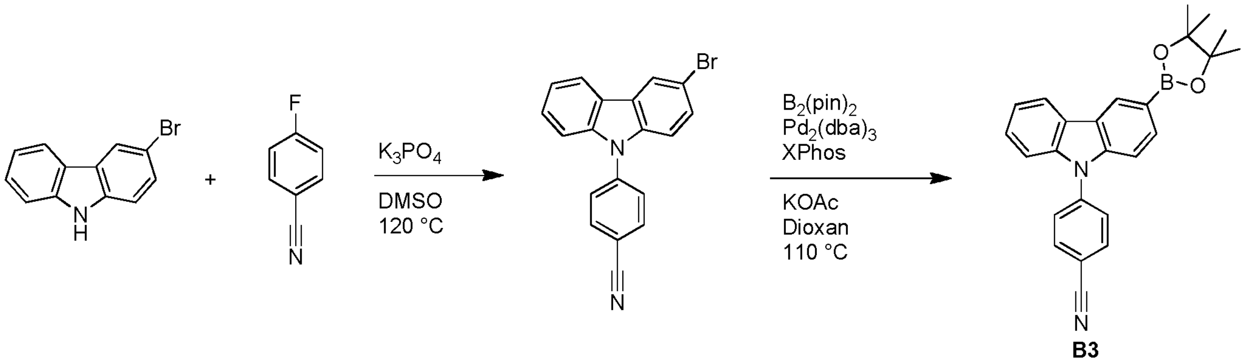


XPhos: CAS-Nummer 564483-18-7
$B_2(pin)_2$: CAS-Nummer: 73183-34-3

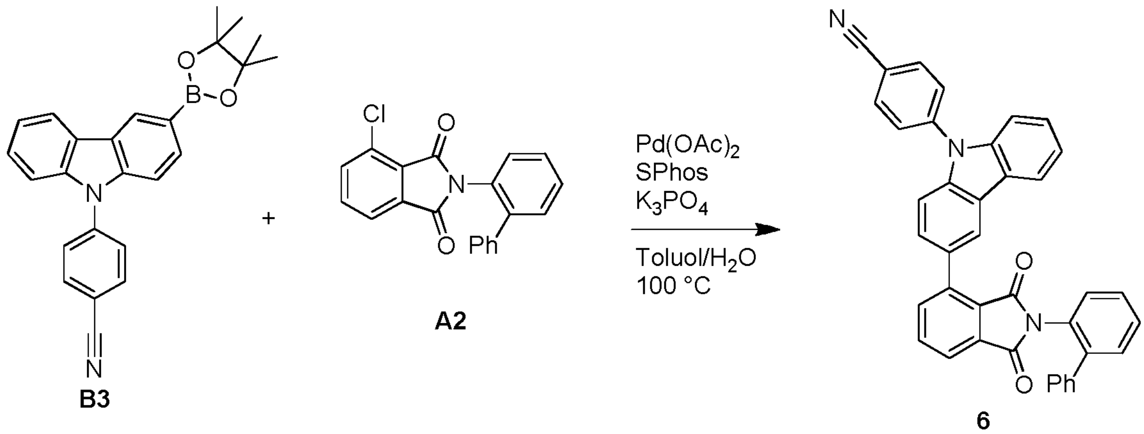


1 Äquivalent **A2** werden mit 1,3 Äquivalenten **B3** analog den Reaktionsbedingungen der Beispiele 1 und 2 umgesetzt. Die Verbindung **6** wird als Feststoff erhalten (Ausbeute 68 %).

Für 6 wurde eine Photolumineszenzquantenausbeute (PLQY) von 55 % (Film 10 % in PMMA) und ein Emissionsmaximum von 472 nm bestimmt. Das Emissionsspektrum ist in Figur 7 dargestellt.

Beispiel 7

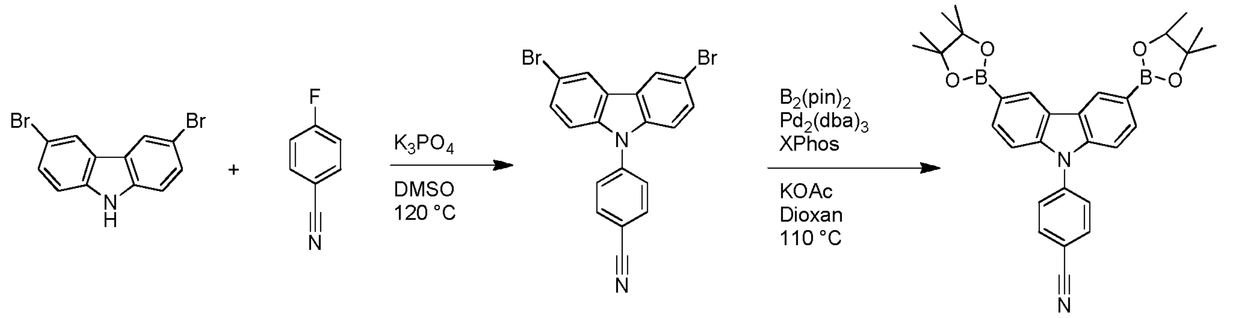


**B4**

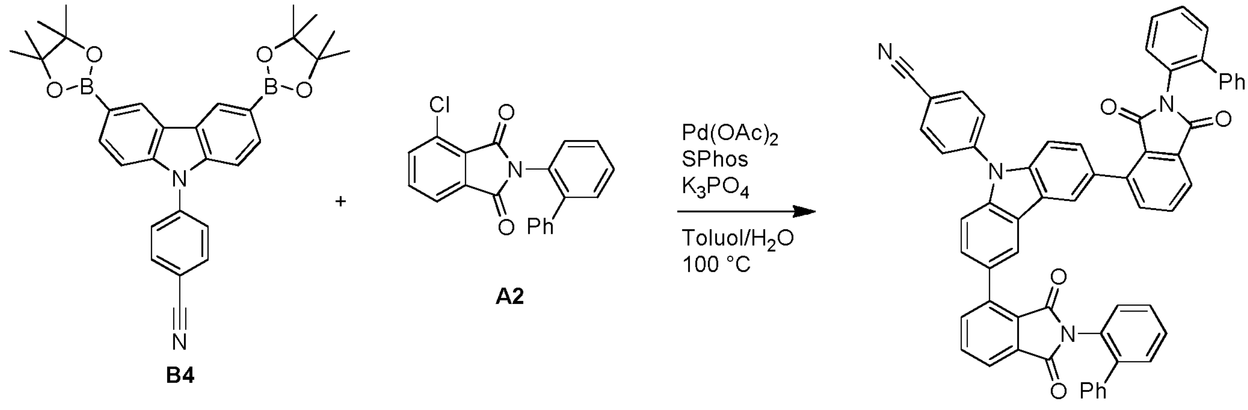


**B4** **A2** **7**

2,5 Äquivalente **A2** werden mit einem Äquivalent **B4** analog den Reaktionsbedingungen der Beispiele 1 und 2 umgesetzt. Die Verbindung **7** wird als Feststoff erhalten (Ausbeute 61 %).

Für 7 wurde eine Photolumineszenzquantenausbeute (PLQY) von 50 % (Film 10 % in PMMA) und ein Emissionsmaximum von 475 nm bestimmt. Das Emissionsspektrum ist in Figur 8 dargestellt.

**Beispiel 8**

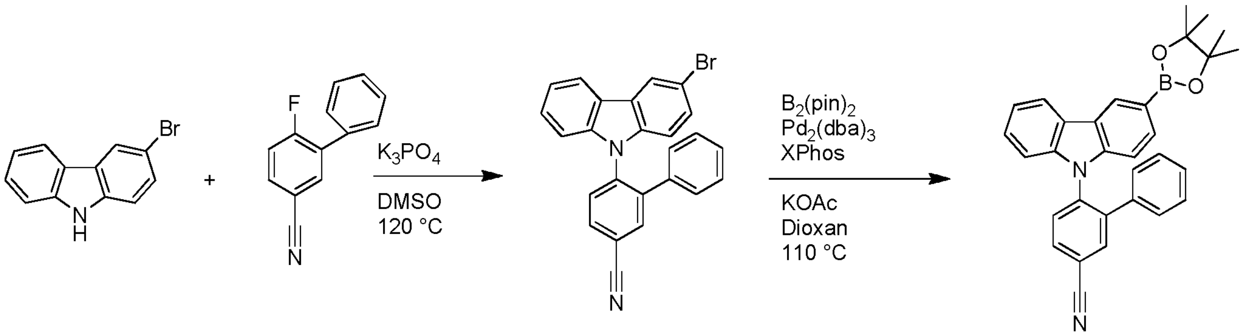


**B5**

XPhos: CAS-Nummer 564483-18-7

$B_2(pin)_2$: CAS-Nummer: 73183-34-3

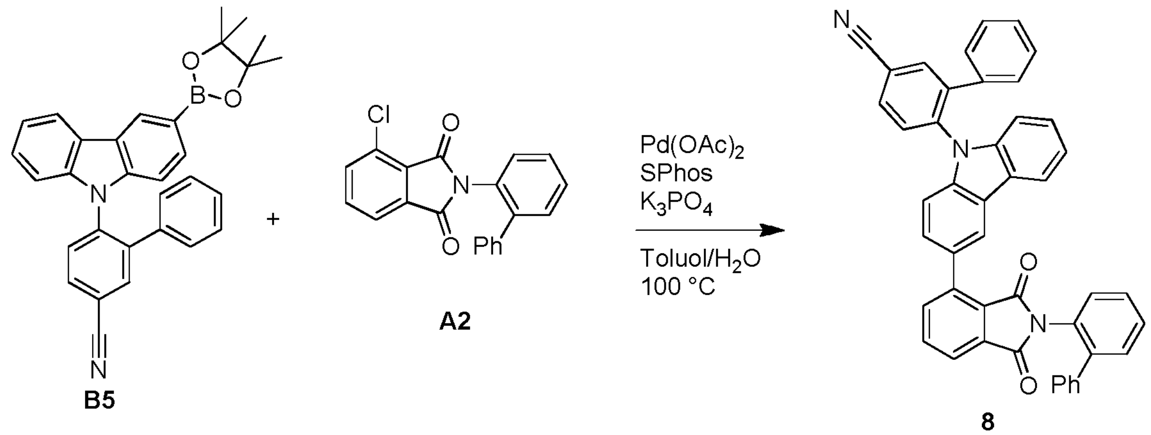


1 Äquivalent **A2** werden mit 1,3 Äquivalenten **B5** analog den Reaktionsbedingungen der Beispiele 1 und 2 umgesetzt. Die Verbindung **8** wird als Feststoff erhalten (Ausbeute 56 %).

Für **8** wurde eine Photolumineszenzquantenausbeute (PLQY) von 58 % (Film 10 % in PMMA) und ein Emissionsmaximum von 464 nm bestimmt. Das Emissionsspektrum ist in Figur 9 dargestellt.

**Beispiel D1:**

Beispiel **1** wurde in einer OLED mit folgendem Aufbau getestet:

| Schicht | Dicke | D1 |
|---|---|---|
| **9** | 100 nm | Al |
| **8** | 2 nm | Liq |
| **7** | 40 nm | NBPhen |
| **6** | 20 nm | **1**:mCBP 20:80 |
| **5** | 10 nm | CzSi |
| **4** | 30 nm | TCTA |
| **3** | 5 nm | NPB |
| **2** | 50 nm | m-MTDATA |
| **1** | 130 nm | ITO |
| **Substrat** | | Glass |

| OLED Nr | Maximale Leistungseffizienz (Power Efficiency) (lm/W) | Maximale Stromausbeute (Current Efficiency) (cd/A) | Maximale EQE (%) | $\lambda_{max}$ (nm) |
|---|---|---|---|---|
| **D1** | 16,9 ± 0,4 | 17,3 ± 0,5 | 7,5 ± 0,2 | 492 |

**Figuren**

**Figur 1:** Schematischer Aufbau eines OLED-Devices.
**Figur 2:** Filmemission von **1** (10 % in PMMA).
**Figur 3:** Filmemission von **2** (10 % in PMMA).
**Figur 4:** Filmemission von **3** (10 % in PMMA).
**Figur 5:** Filmemission von **4** (10 % in PMMA).
**Figur 6:** Filmemission von **5** (10 % in PMMA).
**Figur 7:** Filmemission von **6** (10 % in PMMA).
**Figur 8:** Filmemission von **7** (10 % in PMMA).

**Figur 9:** Filmemission von **7** (10 % in PMMA).
**Figur 10:** Stromeffizienz des OLED-Bauteils **D1.**
**Figur 11:** Stromdichte und Leuchtdichte des OLED-Bauteils **D1.**
**Figur 12:** Externe Quanteneffizienz des OLED-Bauteils **D1.**
**Figur 13:** Elektrolumineszenzspektrum des OLED-Bauteils **D1.**

## Patentansprüche

**1.** Organisches Molekül, aufweisend eine Struktur der Formel A

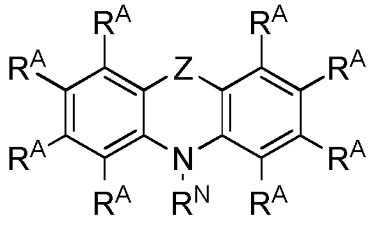

Formel A

mit

Z = eine direkte Bindung oder eine divalente organische Brücke, die eine substituierte oder unsubstituierte C1-C9-Alkylen-, C2-C8-Alkenylen-, C2-C8-Alkinylen- oder Arylen-Gruppe oder eine Kombination dieser, oder -CRR', -C=CRR', -C=NR, -NR-, -O-, SiRR'-, -S-, -S(O)-, $-S(O)_2-$, oder eine durch O unterbrochene substituierte oder unsubstituierte C1-C9-Alkylen-, C2-C8-Alkenylen-, C2-C8-Alkinylen- oder Arylen-Gruppe, oder Phenyl- oder substituierte Phenyleinheiten ist;
$R^N$: bei jedem Auftreten gleich oder verschieden ausgewählt aus der Gruppe bestehend aus:

- H;
- Deuterium;
- einer linearen Alkyl- oder Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;
$R^A$ = bei jedem Auftreten gleich oder verschieden ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann;
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;

und

- einer Gruppe gemäß der Formel B:

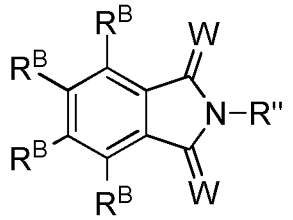

Formel B

wobei

W unabhängig voneinander ausgewählt ist aus der Gruppe bestehend aus $C(CN)_2$, $NR^2$, O und S;
jedes R und R' bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;

- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten R und R' auch miteinander ein mono- oder polycyclisches, aliphatisches, aromatisches und/oder benzoannelliertes Ringsystem bilden;
R" bei jedem Auftreten gleich oder verschieden ist und die gleiche Bedeutung wie $R^N$ hat oder eine Einfachbindung oder eine organische Brücke B*, ist, worüber eine zweite gleiche oder verschiedene Einheit der Formel B verbunden ist, an die genau eine weitere gleiche oder verschiedene Einheit der Formel A gebunden ist, und eine dimere Struktur ausgebildet wird; wobei B* eine substituierte oder unsubstituierte Alkyl- (linear, verzweigt oder zyklisch), Heteroalkyl-, Aryl-, Heteroaryl- oder Alkenyl-Gruppe, insbesondere eine optional substituierte Phenyleinheit ist;
$R^2$ bei jedem Auftreten gleich oder verschieden ausgewählt ist aus der Gruppe bestehend aus:

- H, Deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$;
- einer linearen Alkyl-, Alkoxy- oder Thioalkoxygruppe mit 1 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^3$ substituiert sein kann;
- einer Aryloxy- oder Heteroaryloxygruppe mit 5 bis 60 aromatischen Ringatomen, die mit einem oder mehreren Resten $R^3$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^3$ substituiert sein kann;

dabei können zwei oder mehrere dieser Substituenten $R^2$ auch miteinander ein mono- oder polycyclisches, aromatisches und/oder benzoannelliertes Ringsystem bilden;
$R^3$ bei jedem Auftreten gleich oder verschieden ist H, Deuterium, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;
jedes $R^B$ bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Grup-

pen durch $R^2C{=}CR^2$, C≡C, $Si(R^2)_2$, $Ge(R^2)_2$, $Sn(R^2)_2$, C=O, C=S, C=Se, $C{=}NR^2$, $P({=}O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C{=}CR^2$, C≡C, $Si(R^2)_2$, $Ge(R^2)_2$, $Sn(R^2)_2$, C=O, C=S, C=Se, $C{=}NR^2$, $P({=}O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C{=}CR^2$, C≡C, $Si(R^2)_2$, $Ge(R^2)_2$, $Sn(R^2)_2$, C=O, C=S, C=Se, $C{=}NR^2$, $P({=}O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;
und wobei genau ein Rest $R^B$ pro Einheit der Formel B eine Einfachbindung darstellt, über die die Gruppe gemäß der Formel B an die Struktur gemäß Formel A bindet;
wobei mindestens ein $R^A$ eine Gruppe gemäß Formel B ist.

**2.** Organisches Molekül nach Anspruch 1, aufweisend eine Struktur der Formel C

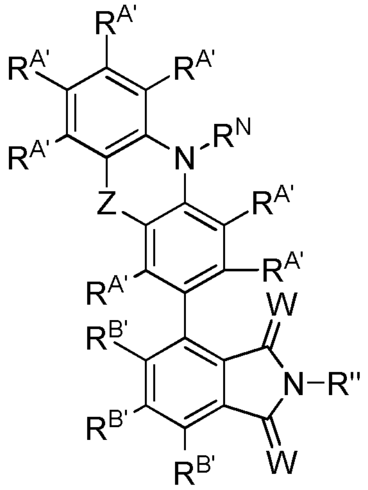

Formel C

wobei

jedes $R^{B'}$ bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C({=}O)R^2$, $P({=}O)(R^2)_2$, $S({=}O)R^2$, $S({=}O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C{=}CR^2$, C=C, $Si(R^2)_2$, $Ge(R^2)_2$, $Sn(R^2)_2$, C=O, C=S, C=Se, $C{=}NR^2$, $P({=}O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;

- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;
$R^{A'}$ = bei jedem Auftreten gleich oder verschieden ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann;
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere der Substituenten $R^2$ auch miteinander ein mono- oder polycyclisches, aliphatisches, aromatisches und/oder benzoannelliertes Ringsystem bilden;

und

- einer Gruppe gemäß der Formel B; wobei maximal ein $R^{A'}$ eine Gruppe gemäß der Formel B darstellt

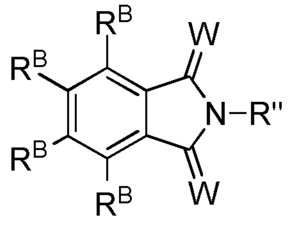

Formel B

und wobei im Übrigen die in Anspruch 1 angegebenen Definitionen gelten.

**3.** Organisches Molekül nach Anspruch 1 oder 2, aufweisend eine Struktur der Formel D

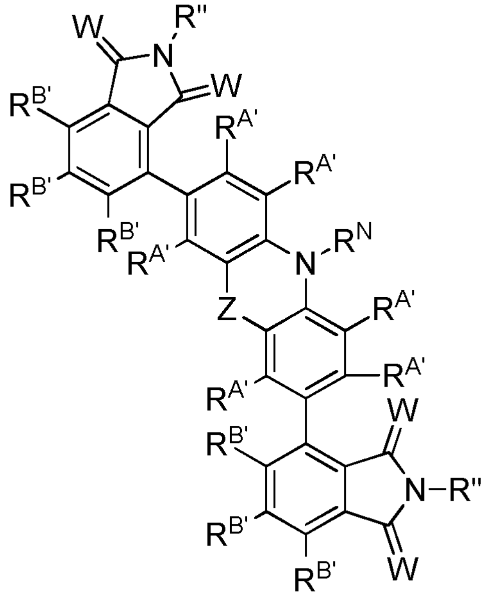

Formel D

wobei

jedes $R^{B'}$ bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das

jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;
$R^{A'}$ = bei jedem Auftreten gleich oder verschieden ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere der Substituenten $R^2$ auch miteinander ein mono- oder polycyclisches, aliphatisches, aromatisches und/oder benzoannelliertes Ringsystem bilden;

und wobei im Übrigen die in Anspruch 1 angegebenen Definitionen gelten.

**4.** Organisches Molekül nach Anspruch 1, aufweisend eine Struktur der Formel E

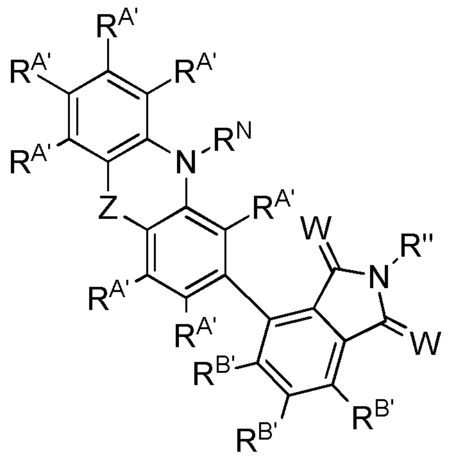

Formel E

wobei

jedes $R^{B'}$ bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann; dabei können zwei oder mehrere dieser Substituenten $R^2$ auch miteinander ein mono- oder polycyclisches, aliphatisches, aromatisches und/oder benzoannelliertes Ringsystem bilden;

$R^{A'}$ = bei jedem Auftreten gleich oder verschieden ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann;
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere der Substituenten $R^2$ auch miteinander ein mono- oder polycyclisches, aliphatisches, aromatisches und/oder benzoannelliertes Ringsystem bilden;

und

- einer Gruppe gemäß der Formel B; wobei maximal ein $R^{A'}$ eine Gruppe gemäß der Formel B darstellt

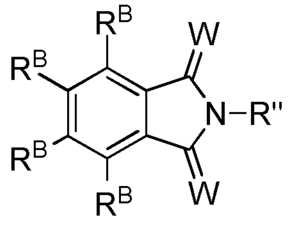

Formel B

und wobei im Übrigen die in Anspruch 1 angegebenen Definitionen gelten.

**5.** Organisches Molekül nach Anspruch 4, aufweisend eine Struktur der Formel F

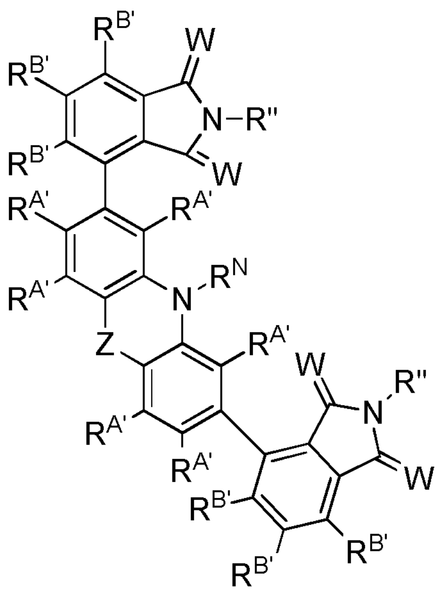

Formel F

wobei

jedes $R^{B'}$ bei jedem Auftreten gleich oder verschieden ausgewählt ist aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten R2 substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten R2 substituiert sein kann, wobei eine oder mehrere

nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann;

dabei können zwei oder mehrere dieser Substituenten $R^2$ auch miteinander ein mono- oder polycyclisches, aliphatisches, aromatisches und/oder benzoannelliertes Ringsystem bilden;
$R^{A'}$ = bei jedem Auftreten gleich oder verschieden ausgewählt aus der Gruppe bestehend aus:

- H, Deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- einer linearen Alkyl-, Alkoxy- oder Thioalkoxygruppe mit 1 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch Deuterium, F, Cl, Br, I, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^2$ substituiert sein kann;
- einer Aryloxy- oder Heteroaryloxygruppe mit 5 bis 60 aromatischen Ringatomen, die mit einem oder mehreren Resten $R^2$ substituiert sein kann;
- einer Diarylaminogruppe, Diheteroarylaminogruppe oder Arylheteroarylaminogruppe mit 10 bis 40 aromatischen Ringatomen, welche mit einem oder mehreren Resten $R^2$ substituiert sein kann; und
- eine Kombination dieser Systeme;

dabei können zwei oder mehrere der Substituenten $R^2$ auch miteinander ein mono- oder polycyclisches, aliphatisches, aromatisches und/oder benzoannelliertes Ringsystem bilden;

und wobei im Übrigen die in Anspruch 1 angegebenen Definitionen gelten.

**6.** Organisches Molekül nach Anspruch 1 bis 5, wobei
W=O,
R" eine Alkyleinheit oder eine Phenyleinheit ist, die optional alkyl-substituiert ist und
Z eine Einfachbindung ist.

**7.** Verwendung eines organischen Moleküls nach Anspruch 1 bis 6 als Emitter und/ oder Host.

**8.** Zusammensetzung aufweisend oder bestehend aus:

(a) mindestens einem organischen Molekül nach einem der Ansprüche 1 bis 6, insbesondere als Emitter und/oder Host, und
(b) optional einer oder mehrerer von dem Molekül nach einem der Ansprüche 1 bis 6 verschiedenen Emitter- und/oder Hostmaterialien
(c) optional eine oder mehreren Farbstoffen und/ oder einem oder mehreren Lösungsmitteln.

**9.** Optoelektronisches Bauelement, aufweisend ein organisches Molekül nach Anspruch 1 bis 6 oder eine Zusammensetzung nach Anspruch 8.

**10.** Optoelektronisches Bauelement nach Anspruch 9, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organisches Molekül nach Anspruch 1 bis 6 oder eine Zusammensetzung nach Anspruch 8 aufweist.

**11.** Verfahren zur Herstellung eines optoelektronischen Bauelements, wobei ein organisches Molekül nach Anspruch 1 bis 6 verwendet wird.

**12.** Verfahren nach Anspruch 11, wobei das organische Molekül durch ein Verdampfungsverfahren oder aus einer Lösung aufgebracht wird.

## Claims

**1.** Organic molecule comprising a structure of formula A

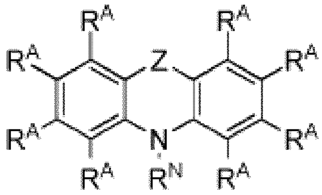

Formula A

with

Z = a direct bond or a divalent organic bridge which is a substituted or unsubstituted C1-C9-alkylene, C2-C8-alkenylene, C2-C8-alkynylene or arylene group or a combination of these, or -CRR', -C=CRR', -C=NR,-NR-, -O-, SiRR'-, -S-, -S(O)-, $-S(O)_2-$, or an O-interrupted substituted or unsubstituted C1-C9-alkylene, C2-C8-alkenylene, C2-C8-alkynylene or arylene group, or phenyl or substituted phenyl units;
$R^N$: identical or different at each instance and selected from the group consisting of:

- H;
- deuterium;
- a linear alkyl or alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each

case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;
$R^A$ = identical or different at each instance and selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;

and

- a group of formula B:

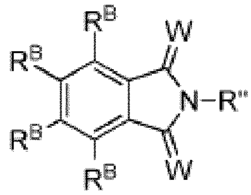

Formula B

where

W is independently selected from the group consisting of $C(CN)_2$, $NR^2$, O and S;
each R and R' is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;

- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents R and R' may also form a mono- or polycyclic, aliphatic, aromatic and/or benzofused ring system with one another;
R" is the same or different at each instance and has the same definition as $R^N$ or is a single bond or an organic bridge B* via which a second identical or different unit of formula B is bonded, to which exactly one further identical or different unit of formula A is bonded, and forms a dimeric structure;
where B* is a substituted or unsubstituted alkyl (linear, branched or cyclic), heteroalkyl, aryl, heteroaryl or alkenyl group, especially an optionally substituted phenyl unit;
$R^2$ is the same or different at each instance and is selected from the group consisting of:

- H, deuterium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$;
- a linear alkyl, alkoxy or thioalkoxy group having 1 to 40 carbon atoms, which may be substituted in each case by one or more $R^3$ radicals, where one or more nonadjacent $CH_2$ groups may be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^3$ radicals, where one or more nonadjacent $CH_2$ groups may be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^3$ radicals, where one or more nonadjacent $CH_2$ groups may be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^3$ radicals;
- an aryloxy or heteroaryloxy group having 5 to 60 aromatic ring atoms, which may be substituted in each case by one or more $R^3$ radicals; and
- a diarylamino group, diheteroarylamino group or arylheteroarylamino group having 10 to 40 aromatic ring atoms, which may be substituted by one or more $R^3$ radicals;

where two or more of these substituents $R^2$ may also form a mono- or polycyclic, aromatic and/or benzofused ring system with one another;
$R^3$ is the same or different at each instance and is H, deuterium, F, $CF_3$ or an aliphatic, aromatic and/or heteroaromatic hydrocarbyl radicals having 1 to 20 carbon atoms, in which one or more hydrogen atoms may also be replaced by F or $CF_3$; it is possible here for two or more substituents $R^3$ to form a mono- or polycyclic aliphatic ring system with one another;
each $R^B$ is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;

- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;
where exactly one $R^B$ radical per unit of formula B represents a single bond via which the group of formula B binds to the structure of formula A;
where at least one $R^A$ is a group of formula B.

**2.** Organic molecule according to Claim 1, comprising a structure of formula C

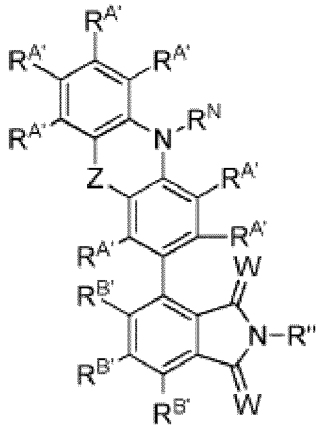

Formula C

where

each $R^{B'}$ is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C,

$Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;
$R^{A'}$ = identical or different at each instance and selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of the substituents $R^2$ may also form a mono- or polycyclic, aliphatic, aromatic and/or benzofused ring system with one another;

and

- a group of formula B; where not more than one $R^{A'}$ represents a group of formula B

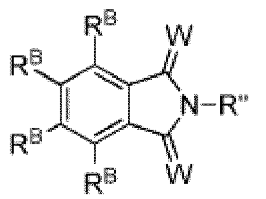

Formula B

and where the definitions specified in Claim 1 are otherwise applicable.

**3.** Organic molecule according to Claim 1 or 2, comprising a structure of formula D

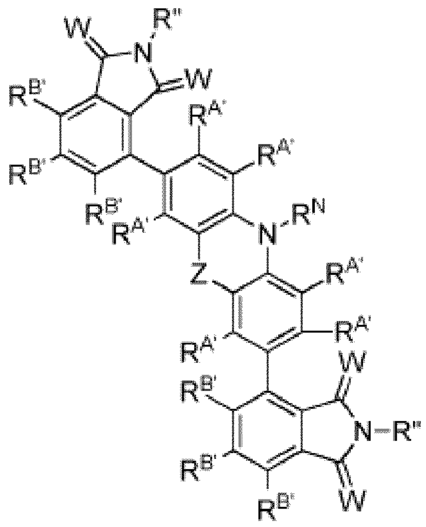

Formula D

where

each $R^{B'}$ is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;
$R^{A'}$ = identical or different at each instance and selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;

- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of the substituents $R^2$ may also form a mono- or polycyclic, aliphatic, aromatic and/or benzofused ring system with one another;

and where the definitions specified in Claim 1 are otherwise applicable.

**4.** Organic molecule according to Claim 1, comprising a structure of formula E

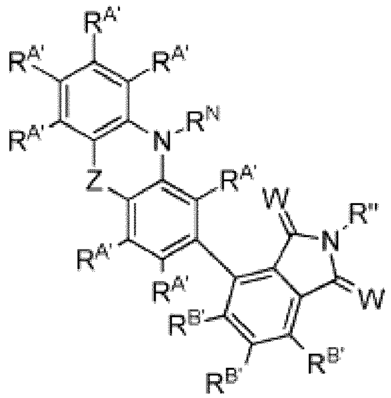

Formula E

where

each $R^{B'}$ is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;

- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals; where two or more of these substituents $R^2$ may also form a mono- or polycyclic, aliphatic, aromatic and/or benzofused ring system with one another;

$R^{A'}$ = identical or different at each instance and selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted in each case by one or more $R^2$ radicals; and
- a diarylamino group, diheteroarylamino group or arylheteroarylamino group having 10 to 40 aromatic ring atoms, which may be substituted by one or more $R^2$ radicals;

where two or more of the substituents $R^2$ may also form a mono- or polycyclic, aliphatic, aromatic and/or benzofused ring system with one another;

and

- a group of formula B; where not more than one $R^{A'}$ represents a group of formula B

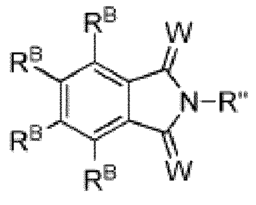

Formula B

and where the definitions specified in Claim 1 are otherwise applicable.

**5.** Organic molecule according to Claim 4, comprising a structure of formula F

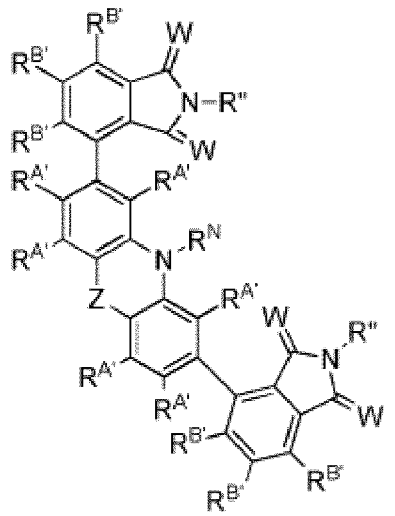

Formula F

where

each $R^{B'}$ is the same or different at each instance and is selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted by one or more $R^2$ radicals; and
- a diarylamino group, diheteroarylamino group or arylheteroarylamino group having 10 to 40 aromatic ring atoms, which may be substituted by one or more $R^2$ radicals;

where two or more of these substituents $R^2$ may also form a mono- or polycyclic, aliphatic, aromatic and/or benzofused ring system with one another;
$R^{A'}$ = identical or different at each instance and selected from the group consisting of:

- H, deuterium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$;
- a linear alkyl, alkoxy or thioalkoxy group having 1 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a linear alkenyl or alkynyl group having 2 to 40 carbon atoms, which may be substituted in each case by one or more $R^2$ radicals, where one or more nonadjacent $CH_2$ groups may be replaced by $R^2C=CR^2$, C=C,

$Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- a branched or cyclic alkyl, alkenyl, alkynyl, alkoxy or thioalkoxy group having 3 to 40 carbon atoms, which may be substituted in each case by one or more $R^2$ radicals, where one or more nonadjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more hydrogen atoms may be replaced by deuterium, F, Cl, Br, I, CN, $CF_3$ or $NO_2$;
- an aromatic or heteroaromatic ring system having 5 to 60 aromatic ring atoms, which may be substituted in each case by one or more $R^2$ radicals;
- an aryloxy or heteroaryloxy group having 5 to 60 aromatic ring atoms, which may be substituted by one or more $R^2$ radicals; and
- a diarylamino group, diheteroarylamino group or arylheteroarylamino group having 10 to 40 aromatic ring atoms, which may be substituted by one or more $R^2$ radicals;
- a combination of these systems;

where two or more of the substituents $R^2$ may also form a mono- or polycyclic, aliphatic, aromatic and/or benzofused ring system with one another;

and where the definitions specified in Claim 1 are otherwise applicable.

**6.** Organic molecule according to Claims 1 to 5, wherein
W = O,
R" is an alkyl unit or a phenyl unit optionally having alkyl substitution and Z is a single bond.

**7.** Use of an organic molecule according to Claims 1 to 6 as an emitter and/or a host.

**8.** Composition comprising or consisting of:

(a) at least one organic molecule according to any of Claims 1 to 6, especially as emitter and/or host, and
(b) optionally one or more emitter and/or host materials other than the molecule according to any of Claims 1 to 6,
(c) optionally one or more dyes and/or one or more solvents.

**9.** Optoelectronic component including an organic molecule according to Claims 1 to 6 or a composition according to Claim 8.

**10.** Optoelectronic component according to Claim 9, comprising

- a substrate,
- an anode and
- a cathode, where the anode or the cathode is applied to the substrate, and
- at least one light emitting layer which is disposed between the anode and the cathode and which includes the organic molecule according to Claims 1 to 6 or a composition according to Claim 8.

**11.** Process for producing an optoelectronic component, wherein an organic molecule according to Claims 1 to 6 is used.

**12.** Process according to Claim 11, wherein the organic molecule is applied by an evaporation method or from a solution.

**Revendications**

**1.** Molécule organique, présentant une structure de formule A

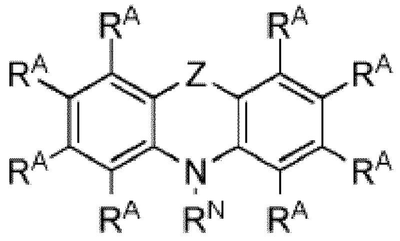

formule A

avec

Z = une liaison directe ou un pont organique divalent, qui est un groupe $C_{1-9}$-alkylène, $C_{2-8}$-alcénylène, $C_{2-8}$-alcynylène ou arylène, substitué ou non substitué, ou une combinaison de ceux-ci, ou-CRR', -C=CRR', -C=NR, -NR-, -O-, SiRR'-, -S-, -S(O)-, $-S(O)_2-$, ou un groupe $C_{1-9}$-alkylène, $C_{2-8}$-alcénylène, $C_{2-8}$-alcynylène ou arylène, substitué ou non substitué, interrompu par O, ou des motifs phényle ou des motifs phényle substitués ;
$R^N$ : en chaque occurrence identique ou différent, étant choisi dans le groupe constitué par :

- H;
- deutérium ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
$R^A$ = en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$

ou $NO_2$;

- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;

et

- un groupe selon la formule B :

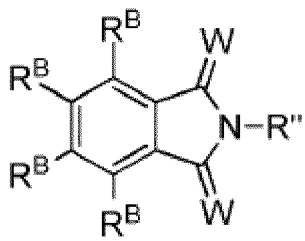

formule B

W, indépendamment les uns des autres, étant choisis dans le groupe constitué par $C(CN)_2$, $NR^2$, O et S ;
chaque R et R', identiques ou différents en chaque occurrence, étant choisis dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants R et R' pouvant également former l'un avec l'autre un système cyclique monocyclique ou polycyclique, aliphatique, aromatique et/ou benzo-annelé ;
R" étant en chaque occurrence identique ou différent et possédant la même signification que $R^N$ ou étant une simple liaison ou un pont organique B*, par l'intermédiaire duquel un deuxième motif identique ou différent de formule B est relié, auquel exactement un autre motif identique ou différent de formule A est lié, et une structure dimère étant formée ; B* étant un groupe alkyle (linéaire, ramifié ou cyclique), hétéroalkyle, aryle, hétéroaryle ou alcényle, substitué ou non substitué, en particulier étant un motif phényle éventuellement substitué ;
$R^2$, en chaque occurrence identique ou différent, étant choisi dans le groupe constitué par :

- H, deutérium, F, Cl, Br, I, $N(R^3)_2$, CN, $CF_3$, $NO_2$, OH, COOH, $COOR^3$, $CO(NR^3)_2$, $Si(R^3)_3$, $B(OR^3)_2$, $C(=O)R^3$, $P(=O)(R^3)_2$, $S(=O)R^3$, $S(=O)_2R^3$, $OSO_2R^3$;
- un groupe alkyle ou alcoxy ou thioalcoxy linéaire comportant 1 à 40 atomes de C, qui à chaque fois peut être substitué par un ou plusieurs radicaux $R^3$, un ou plusieurs groupes $CH_2$ non voisins pouvant être remplacés par $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^3$, un ou plusieurs groupes $CH_2$ non voisins pouvant être remplacés par $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^3$, un ou plusieurs groupes $CH_2$ non voisins pouvant être remplacés par $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^3$ ;
- un groupe aryloxy ou hétéroaryloxy comportant 5 à 60 atomes de cycle aromatiques, qui peut être substitué par un ou plusieurs radicaux $R^3$ ; et
- un groupe diarylamino, dihétéroarylamino ou arylhétéroarylamino comportant 10 à 40 atomes de cycle aromatiques, qui peut être substitué par un ou plusieurs radicaux $R^3$ ;

à cet égard, deux ou plus de ces substituants $R^2$ pouvant également former l'un avec l'autre un système cyclique monocyclique ou polycyclique, aromatique et/ou benzo-annelé ;
$R^3$, en chaque occurrence identique ou différent, étant H, deutérium, F, $CF_3$, un radical hydrocarboné aliphatique, aromatique et/ou hétéroaromatique comportant 1 à 20 atomes de C, dans lequel également un ou plusieurs atomes de H peuvent être remplacés par F ou $CF_3$ ; à cet égard, deux substituants $R^3$ ou plus pouvant également former l'un avec l'autre un système cyclique aliphatique monocyclique ou polycyclique ;
chaque $R^B$, en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O,

C=S, C=Se, C=N$R^2$, P(=O)($R^2$), SO, $SO_2$, N$R^2$, O, S ou CON$R^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
et exactement un radical $R^B$ par motif de formule B représentant une simple liaison, par l'intermédiaire de laquelle le groupe selon la formule B est lié à la structure selon la formule A ;
au moins un $R^A$ étant un groupe selon la formule B.

**2.** Molécule organique selon la revendication 1, présentant une structure de formule C

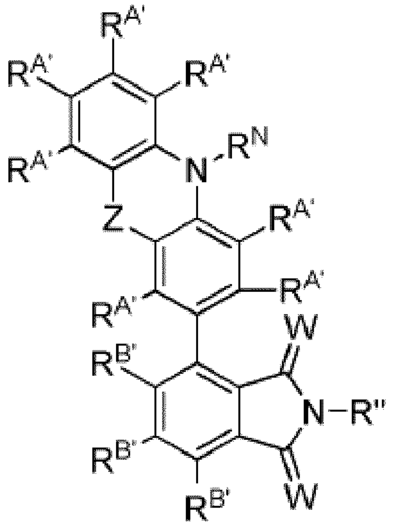

formule C

chaque $R^{B'}$, en chaque occurrence identique ou différent, étant choisi dans le groupe constitué par :

- H, deutérium, F, Cl, Br, I, $N(R^2)_2$, CN, $CF_3$, $NO_2$, OH, COOH, COO$R^2$, $CO(NR^2)_2$, $Si(R^2)_3$, $B(OR^2)_2$, C(=O)$R^2$, P(=O)$(R^2)_2$, S(=O)$R^2$, S(=O)$_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C$=C$R^2$, C=C, $Si(R^2)_2$, $Ge(R^2)_2$, $Sn(R^2)_2$, C=O, C=S, C=Se, C=N$R^2$, P(=O)($R^2$), SO, $SO_2$, N$R^2$, O, S ou CON$R^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C$=C$R^2$, C=C, $Si(R^2)_2$, $Ge(R^2)_2$, $Sn(R^2)_2$, C=O, C=S, C=Se, C=N$R^2$, P(=O)($R^2$), SO, $SO_2$, N$R^2$, O, S ou CON$R^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C$=C$R^2$, C=C, $Si(R^2)_2$, $Ge(R^2)_2$, $Sn(R^2)_2$, C=O, C=S, C=Se, C=N$R^2$, P(=O)($R^2$), SO, $SO_2$, N$R^2$, O, S ou CON$R^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;

- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
$R^{A'}$ = en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus des substituants $R^2$ pouvant également former l'un avec l'autre un système cyclique monocyclique ou polycyclique, aliphatique, aromatique et/ou benzo-annelé ;

et

- un groupe selon la formule B ; au maximum un $R^{A'}$ représentant un groupe selon la formule B

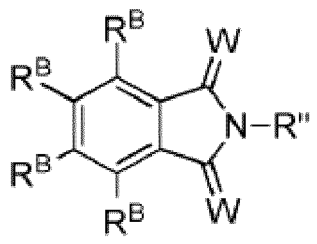

formule B

et pour le reste, les définitions mentionnées dans la revendication 1 s'appliquant.

**3.** Molécule organique selon la revendication 1 ou 2, présentant une structure de formule D

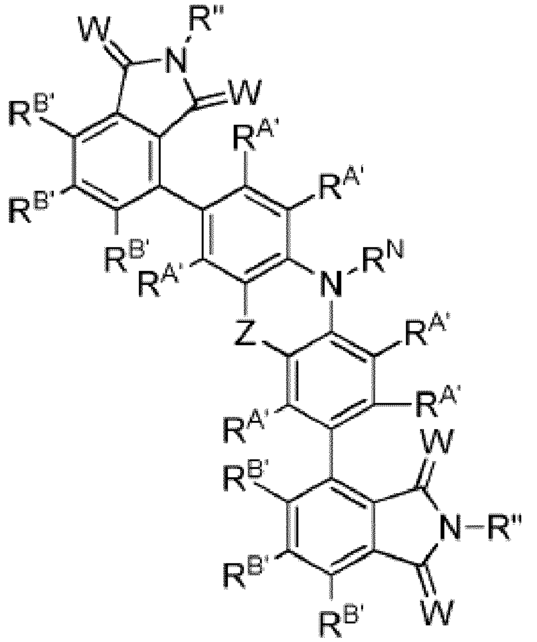

formule D

chaque $R^{B'}$, en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
$R^{A'}$ = en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être

remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;

- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus des substituants $R^2$ pouvant également former l'un avec l'autre un système cyclique monocyclique ou polycyclique, aliphatique, aromatique et/ou benzo-annelé ;
et pour le reste, les définitions mentionnées dans la revendication 1 s'appliquant.

**4.** Molécule organique selon la revendication 1, présentant une structure de formule E

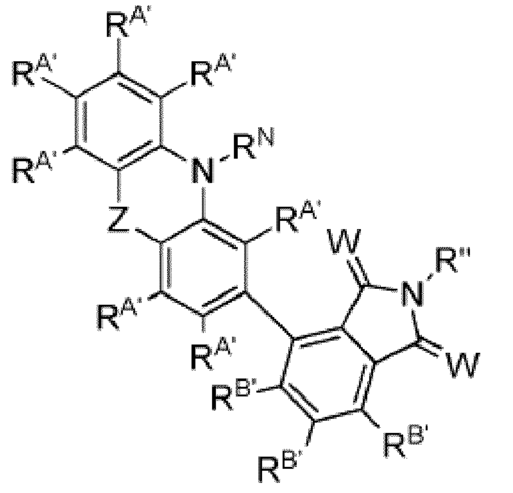

formule E

chaque $R^{B'}$, en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;

- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
$R^{A'}$ = en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus des substituants $R^2$ pouvant également former l'un avec l'autre un système cyclique monocyclique ou polycyclique, aliphatique, aromatique et/ou benzo-annelé ;

et

- un groupe selon la formule B ; au maximum un $R^{A'}$ représentant un groupe selon la formule B

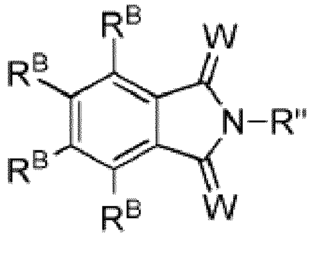

formule B

et pour le reste, les définitions mentionnées dans la revendication 1 s'appliquant.

**5.** Molécule organique selon la revendication 4, présentant une structure de formule F

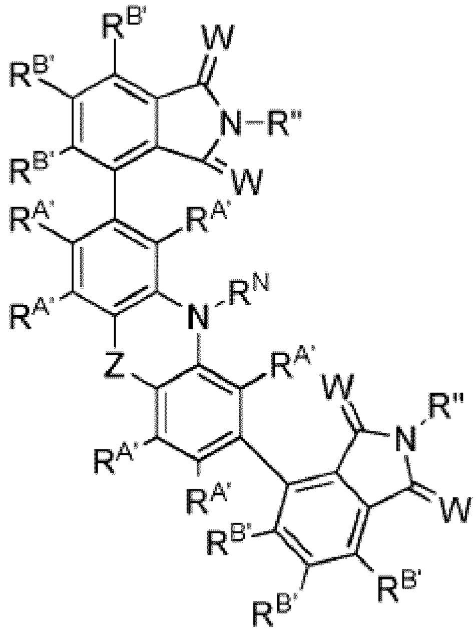

Formel F

chaque $R^{B'}$, en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$, $C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ; et
- un groupe diarylamino, dihétéroarylamino ou arylhétéroarylamino comportant 10 à 40 atomes de cycle aromatiques, qui peut être substitué par un ou plusieurs radicaux $R^2$ ;

à cet égard, deux ou plus de ces substituants $R^2$ pouvant également former l'un avec l'autre un système cyclique monocyclique ou polycyclique, aliphatique, aromatique et/ou benzo-annelé ;
$R^{A'}$ = en chaque occurrence identique ou différent, étant choisi dans le groupe constitué par :

- H, deutérium, F, Cl, Br, I, $N(R^2)_2$, CN, $CF_3$, $NO_2$, OH, COOH, $COOR^2$, $CO(NR^2)_2$, $Si(R^2)_3$, $B(OR^2)_2$,

$C(=O)R^2$, $P(=O)(R^2)_2$, $S(=O)R^2$, $S(=O)_2R^2$, $OSO_2R^2$ ;
- un groupe alkyle ou alcoxy ou thioalcoxy linéaire comportant 1 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alcényle ou alcynyle linéaire comportant 2 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un groupe alkyle, alcényle, alcynyle, alcoxy ou thioalcoxy ramifié ou cyclique comportant 3 à 40 atomes de C, qui à chaque fois peut être substitué par un ou plusieurs radicaux $R^2$, un ou plusieurs groupes $CH_2$ non voisins pouvant être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et un ou plusieurs atomes de H pouvant être remplacés par deutérium, F, Cl, Br, I, CN, $CF_3$ ou $NO_2$ ;
- un système cyclique aromatique ou hétéroaromatique comportant 5 à 60 atomes de cycle aromatiques, qui peut à chaque fois être substitué par un ou plusieurs radicaux $R^2$ ;
- un groupe aryloxy ou hétéroaryloxy comportant 5 à 60 atomes de cycle aromatiques, qui peut être substitué par un ou plusieurs radicaux $R^2$ ;
- un groupe diarylamino, dihétéroarylamino ou arylhétéroarylamino comportant 10 à 40 atomes de cycle aromatiques, qui peut être substitué par un ou plusieurs radicaux $R^2$ ;
- une combinaison de ces systèmes ;

à cet égard, deux ou plus des substituants $R^2$ pouvant également former l'un avec l'autre un système cyclique monocyclique ou polycyclique, aliphatique, aromatique et/ou benzo-annelé ;
et pour le reste, les définitions mentionnées dans la revendication 1 s'appliquant.

**6.** Molécule organique selon les revendications 1 à 5,
W = O,
R" étant un motif alkyle ou un motif phényle, qui est éventuellement substitué par alkyle et
Z étant une simple liaison.

**7.** Utilisation d'une molécule organique selon les revendications 1 à 6 en tant qu'émetteur et/ou hôte.

**8.** Composition présentant ou étant constituée de :

(a) au moins une molécule organique selon l'une quelconque des revendications 1 à 6, en particulier en tant qu'émetteur et/ou hôte, et
(b) éventuellement un ou plusieurs matériaux émetteurs et/ou hôtes différents de la molécule selon l'une quelconque des revendications 1 à 6
(c) éventuellement un ou plusieurs colorants et/ou un ou plusieurs solvants.

**9.** Composant optoélectronique, présentant une molécule organique selon les revendications 1 à 6 ou une composition selon la revendication 8.

**10.** Composant optoélectronique selon la revendication 9, présentant

- un substrat,
- une anode et
- une cathode, l'anode ou la cathode étant déposée sur le substrat, et
- au moins une couche luminescente, qui est disposée entre l'anode et la cathode et qui présente la molécule organique selon les revendications 1 à 6 ou une composition selon la revendication 8.

**11.** Procédé pour la préparation d'un composant optoélectronique, une molécule organique selon les revendications 1 à 6 étant utilisée.

**12.** Procédé selon la revendication 11, la molécule organique étant déposée par un procédé d'évaporation ou à partir

d’une solution.

Figur 1

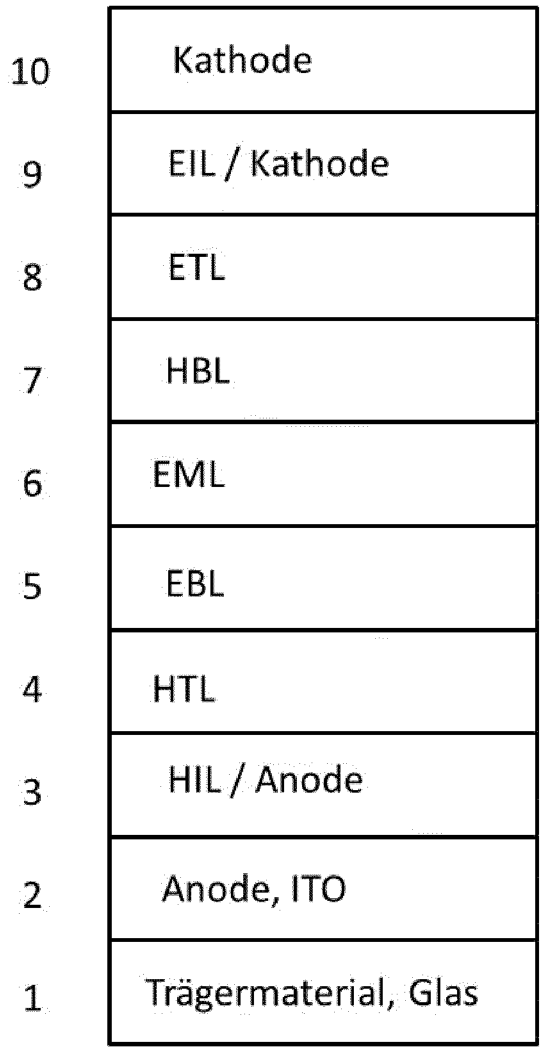
10
Kathode
9
EIL / Kathode
8
ETL
7
HBL
6
EML
5
EBL
4
HTL
3
HIL / Anode
2
Anode, ITO
1
Trägermaterial, Glas

**Figur 2**

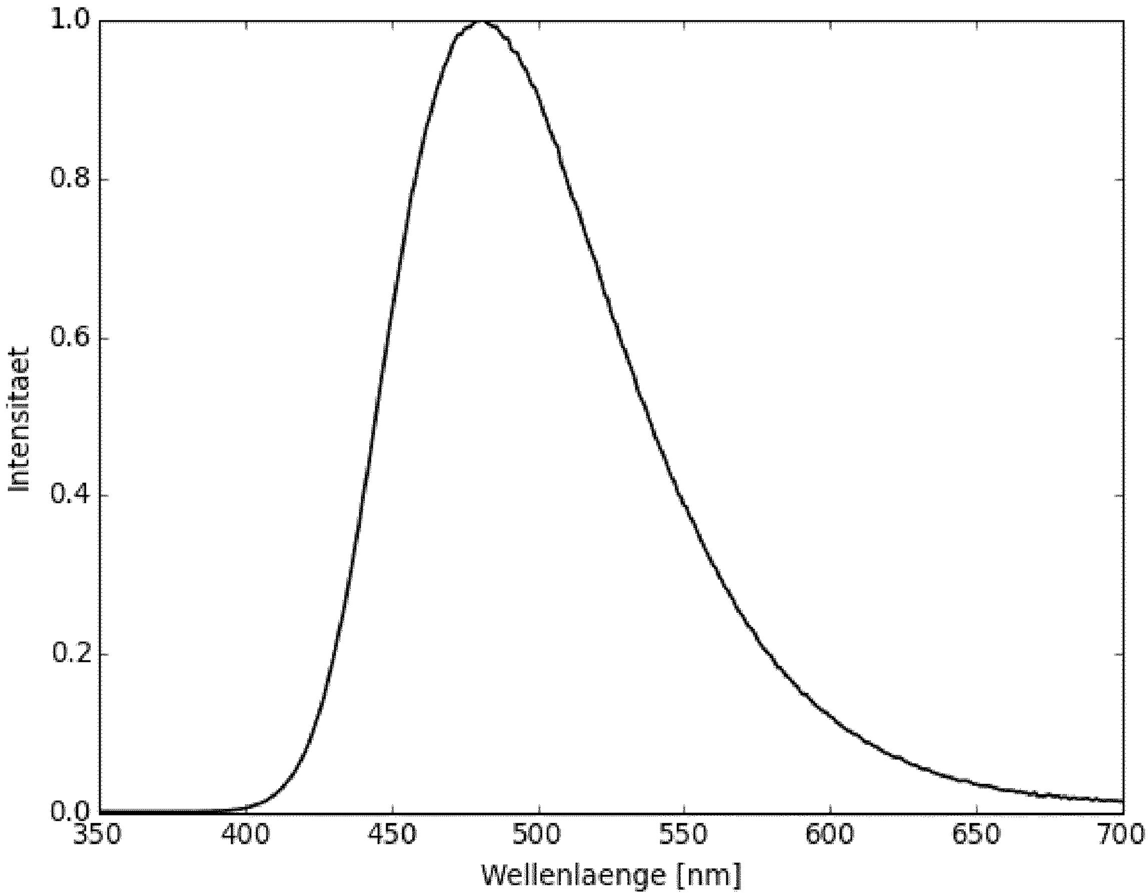
1.0
0.8
0.6
0.4
0.2
0.0
Intensitaet
350
400
450
500
550
600
650
700
Wellenlaenge [nm]

Figur 3

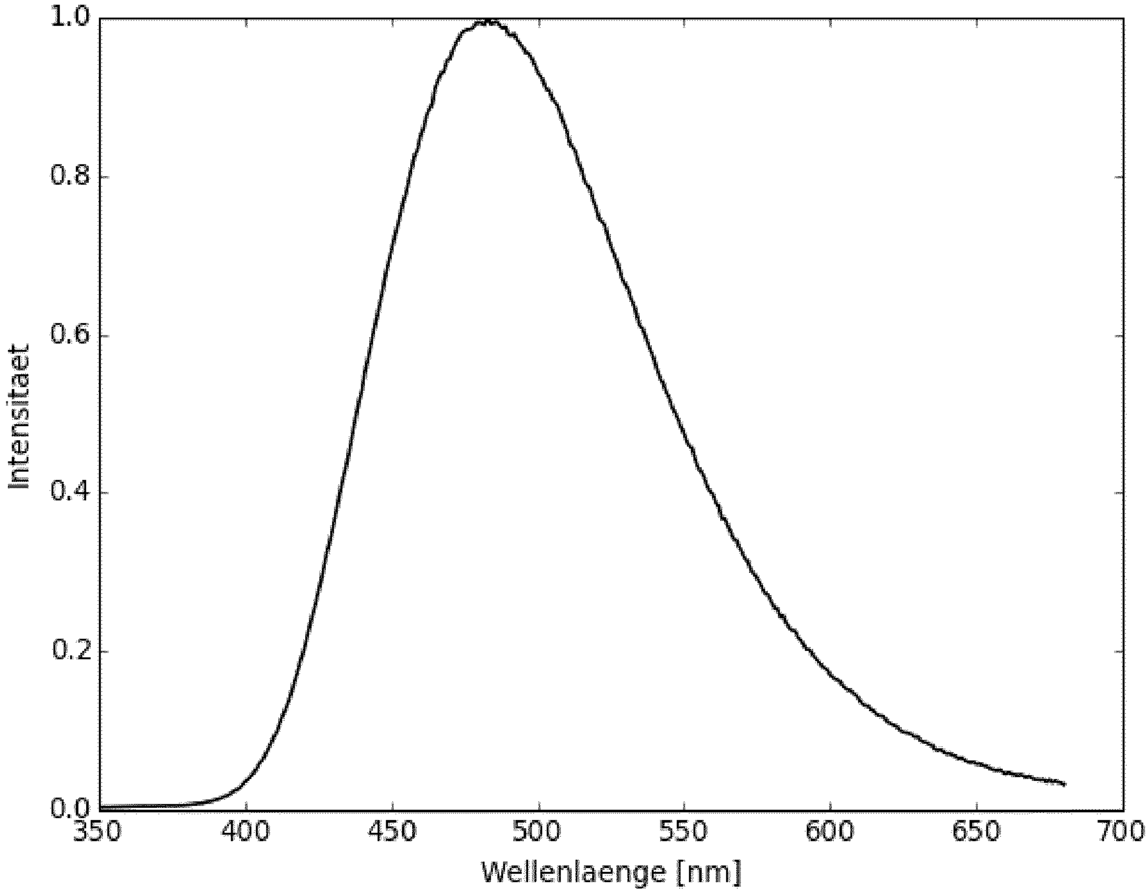
1.0
0.8
0.6
0.4
0.2
0.0
Intensitaet
350
400
450
500
550
600
650
700
Wellenlaenge [nm]

**Figur 4**

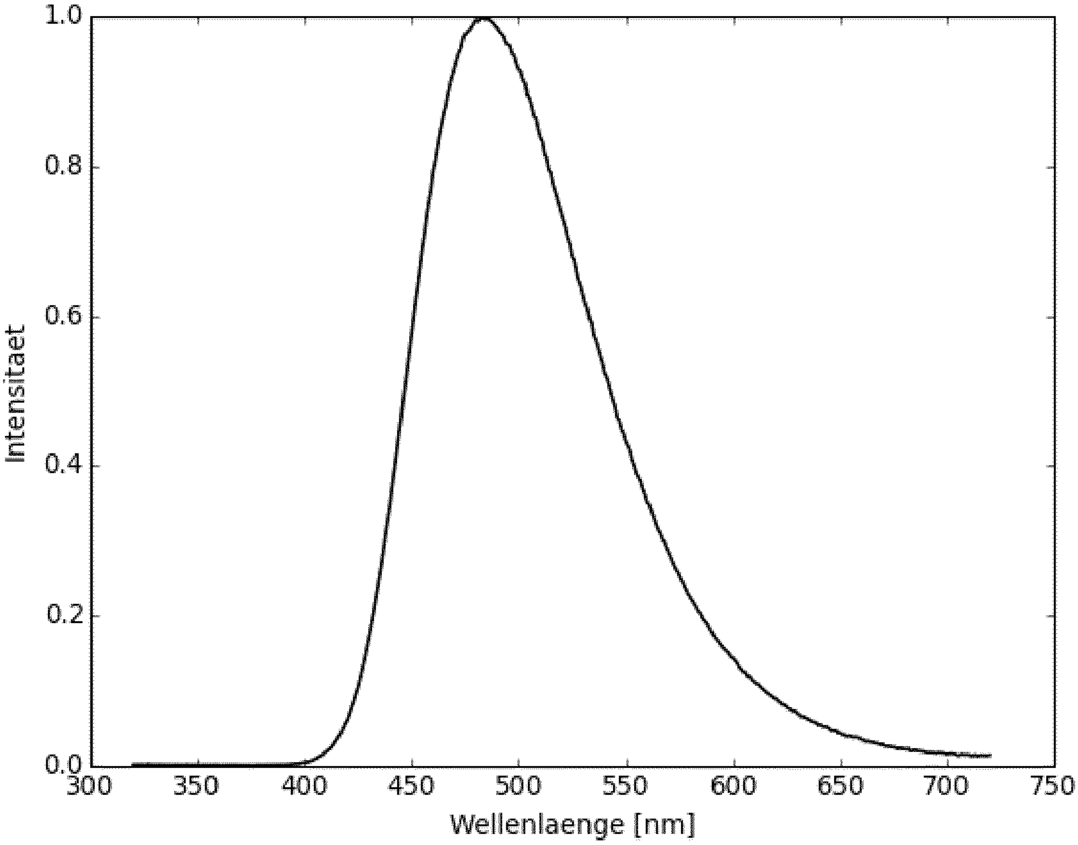
1.0
0.8
0.6
0.4
0.2
0.0
Intensitaet
300
350
400
450
500
550
600
650
700
750
Wellenlaenge [nm]

Figur 5

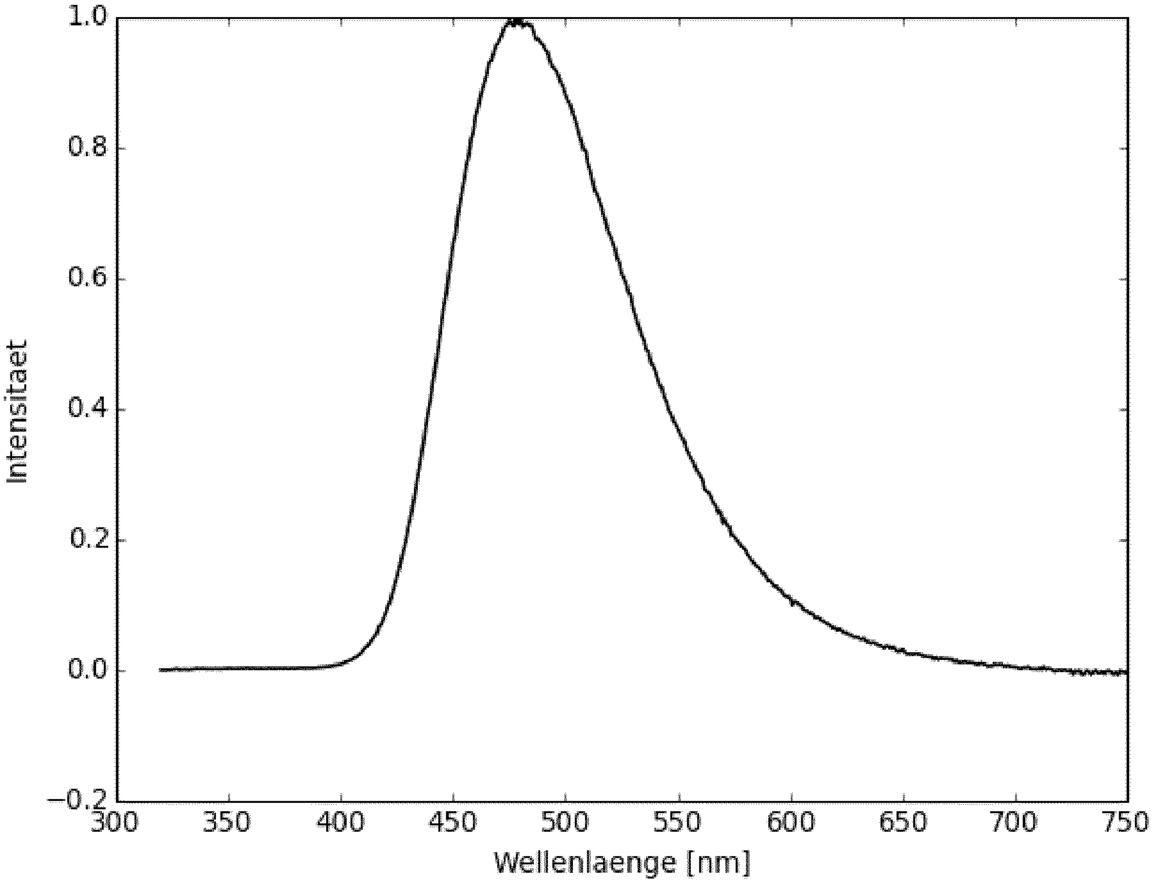
1.0
0.8
0.6
0.4
0.2
0.0
−0.2
Intensitaet
300
350
400
450
500
550
600
650
700
750
Wellenlaenge [nm]

Figur 6

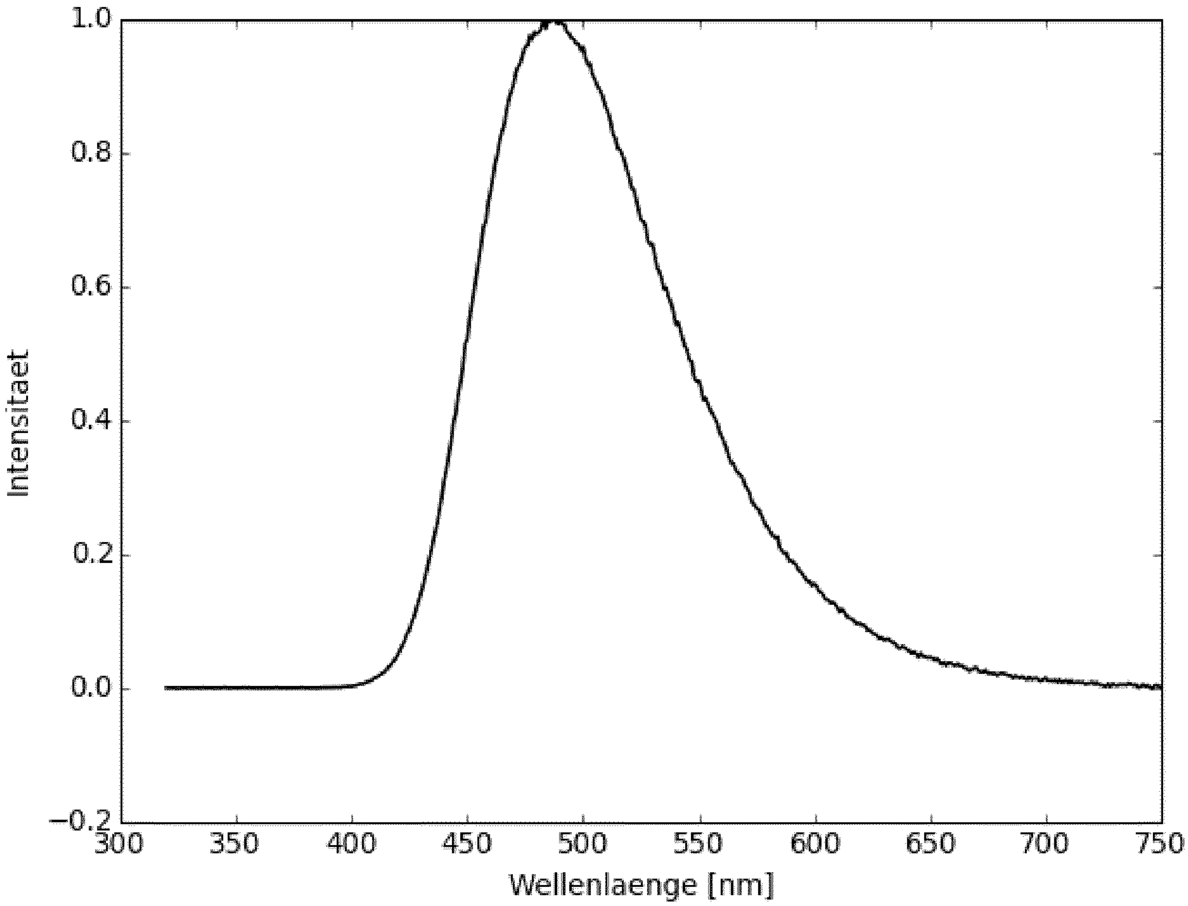
1.0
0.8
0.6
0.4
0.2
0.0
−0.2
Intensitaet
300
350
400
450
500
550
600
650
700
750
Wellenlaenge [nm]

**Figur 7**

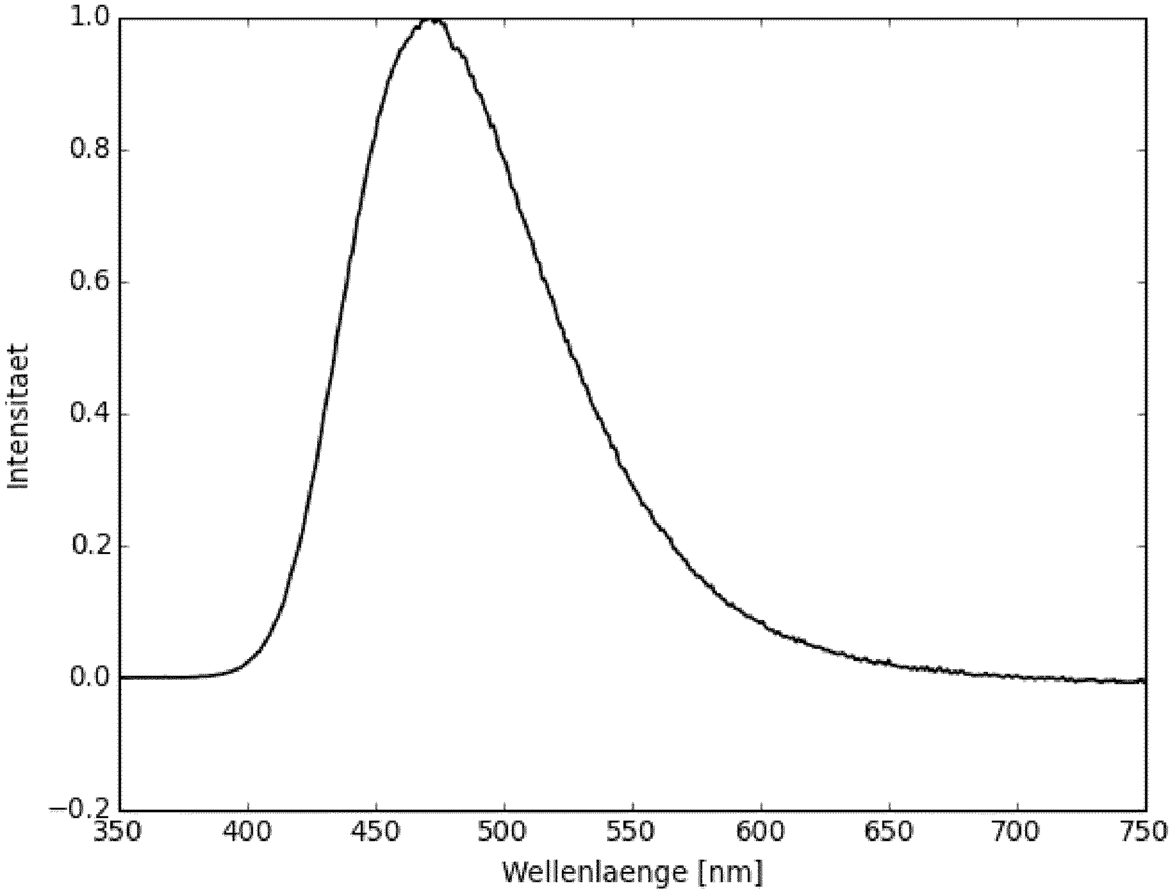
1.0
0.8
0.6
0.4
0.2
0.0
−0.2
Intensitaet
350
400
450
500
550
600
650
700
750
Wellenlaenge [nm]

Figur 8

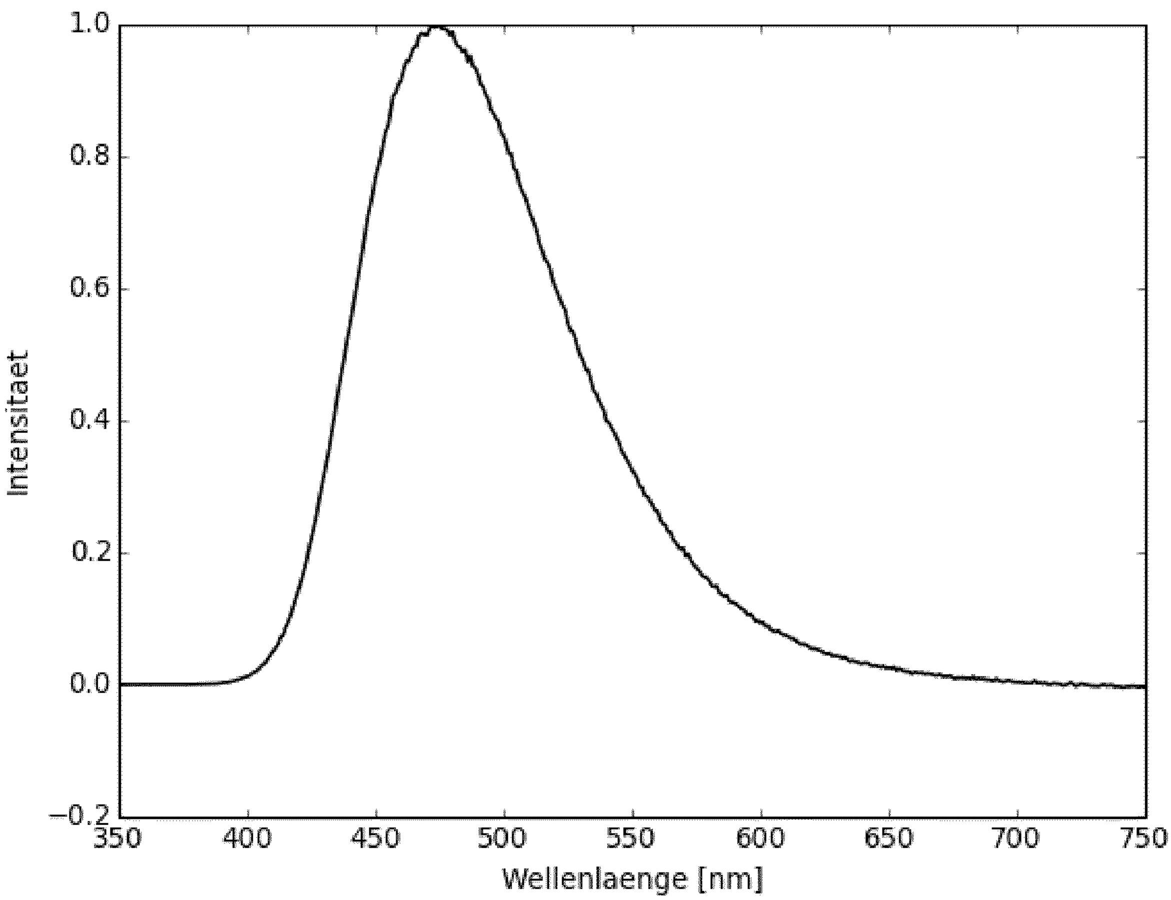
1.0
0.8
0.6
0.4
0.2
0.0
−0.2
Intensitaet
350
400
450
500
550
600
650
700
750
Wellenlaenge [nm]

Figur 9

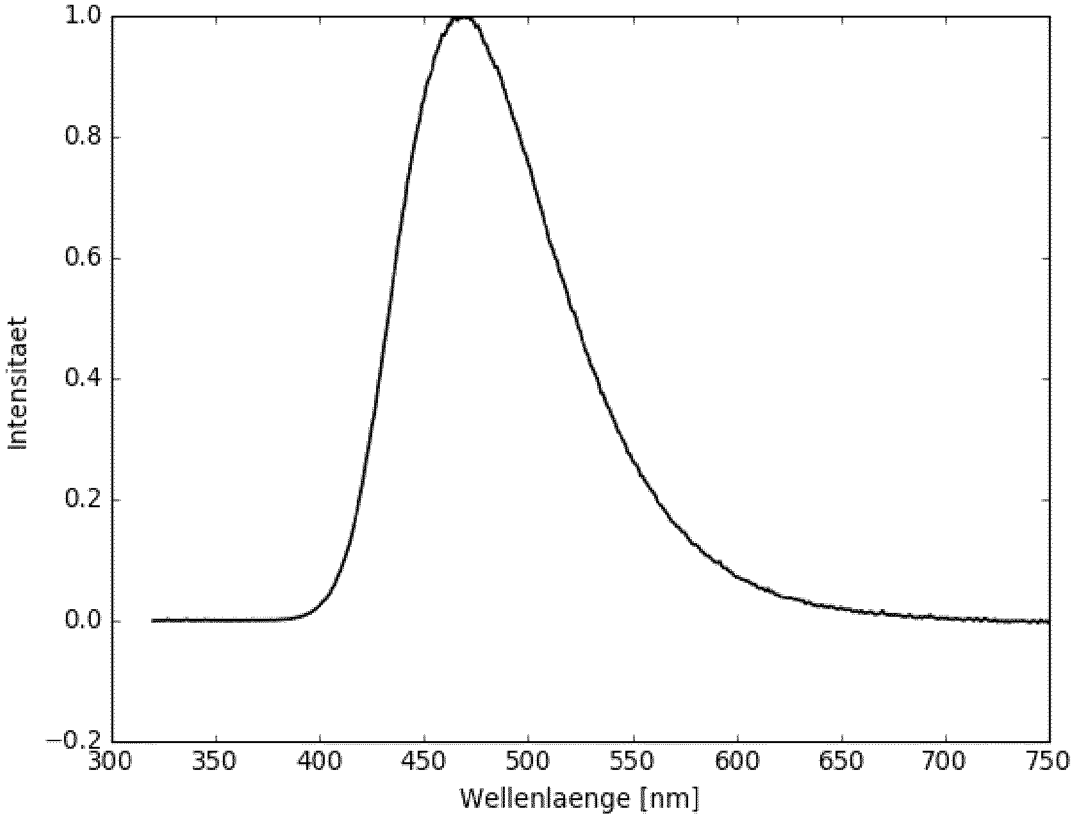
1.0
0.8
0.6
0.4
0.2
0.0
−0.2
Intensitaet
300
350
400
450
500
550
600
650
700
750
Wellenlaenge [nm]

Figur 10

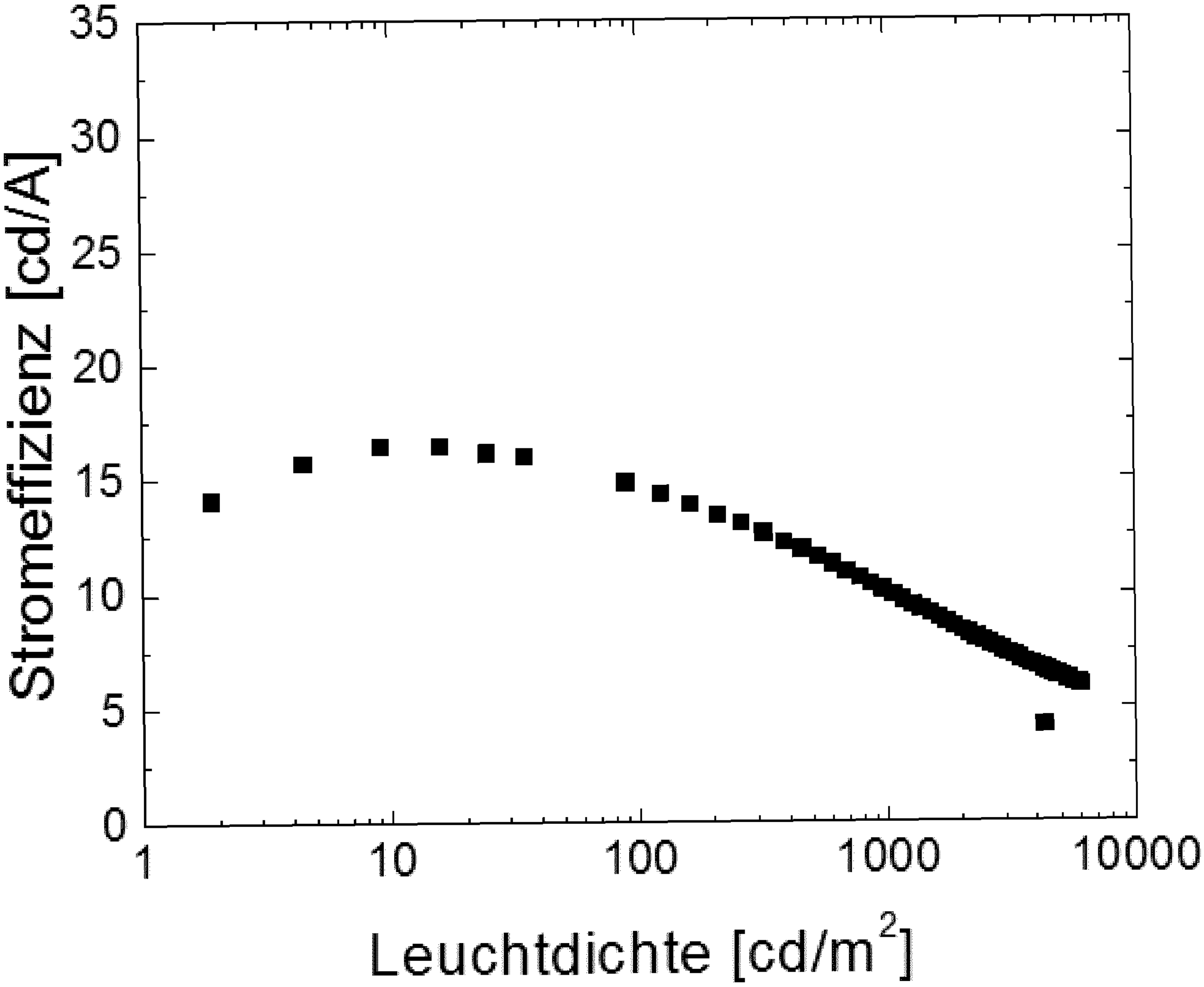

Stromeffizienz [cd/A]
35
30
25
20
15
10
5
0
1
10
100
1000
10000
Leuchtdichte [cd/m2]

Figur 11

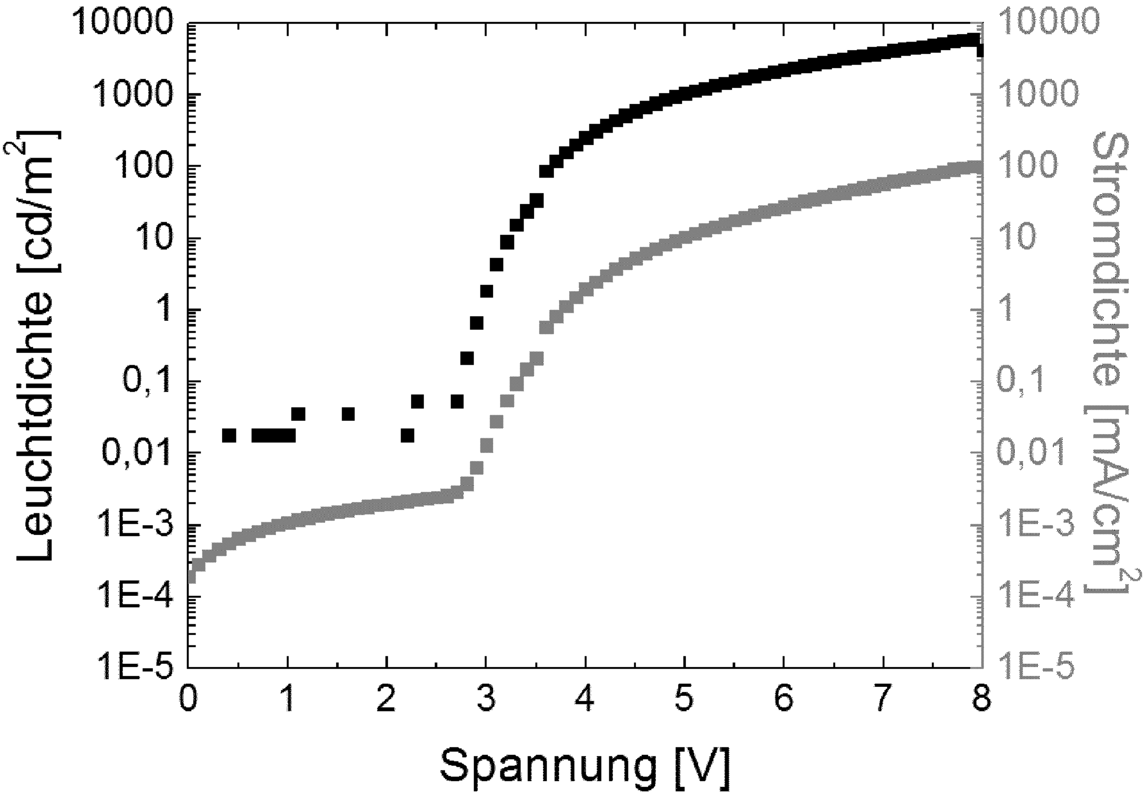
Leuchtdichte [cd/m2]
Stromdichte [mA/cm2]
Spannung [V]
10000
1000
100
10
1
0,1
0,01
1E-3
1E-4
1E-5
0
1
2
3
4
5
6
7
8

Figur 12

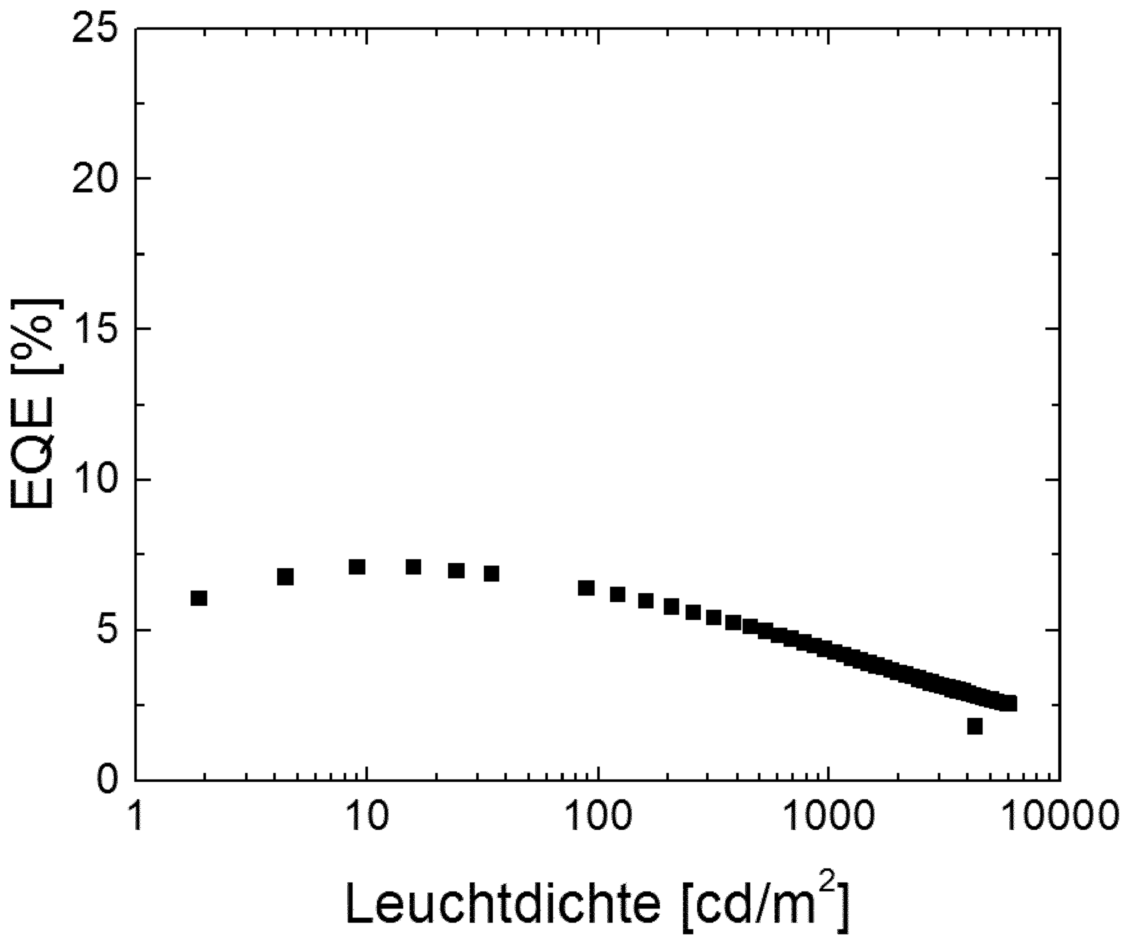

Figur 13

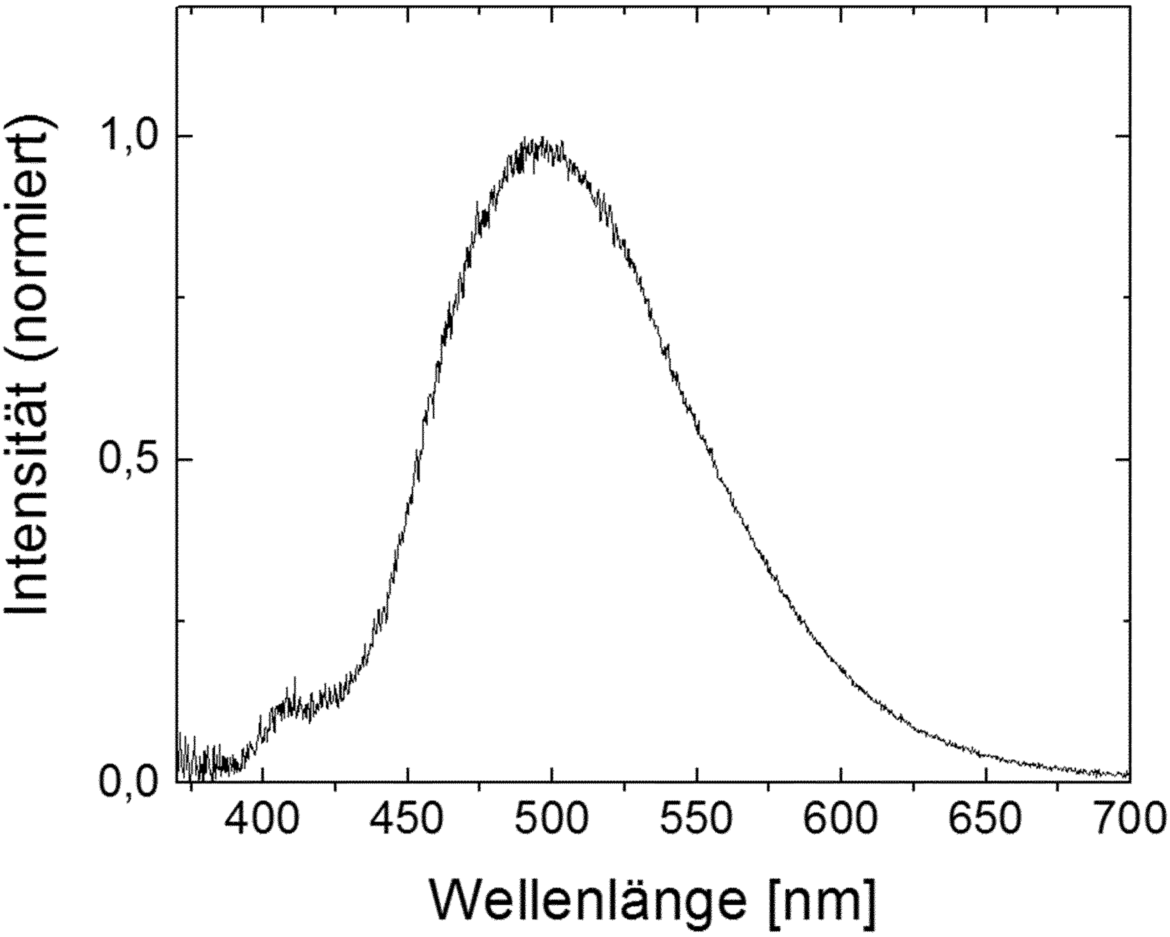
Intensität (normiert)
1,0
0,5
0,0
400
450
500
550
600
650
700
Wellenlänge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **M. A. BALDO ; D. F. O'BRIAN ; M. E. THOMPSON ; S. R. FORREST.** *Phys. Rev. B,* 1999, vol. 60, 14422
- **H. YERSIN.** *Top. Curr. Chem.,* 2004, vol. 241, 1
- **H. UOYAMA ; K. GOUSHI ; K. SHIZU ; H. NOMURA ; C. ADACHI.** *Nature,* 2012, vol. 492, 234
- **KIM, C.W.** *Journal of Nanoscience and Nanotechnology,* 2012, vol. 12 (5), 4219-4223
- **BECKE, A. D.** *Phys. Rev.,* vol. A1988 (38), 3098-3100
- **PERDEW, J. P.** *Phys. Rev. B,* 1986, vol. 33, 8822-8827
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **BECKE, A.D.** *J. Chem. Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11
- **B. M.E. THOMPSON et al.** *Chem. Mater.,* 2004, vol. 16, 4743
- **XIE, Z. T. ; ZHANG, W. H. ; DING, B. F. ; GAO, X. D. ; YOU, Y. T. ; SUN, Z. Y. ; HOU, X. Y.** Interfacial reactions at Al/LiF and LiF/Al. *Applied Physics Letters,* 2009, vol. 94 (6), 063302